# EUROPEAN PATENT SPECIFICATION

(11) **EP 4 370 191 B1**
(45) Date of publication and mention of the grant of the patent: **11.11.2026**
(21) Application number: 22843047.6
(22) Date of filing: 14.07.2022
(51) Int. Cl.: A61M 25/10

(54) **MULTI-FUNCTION CATHETER**
MULTIFUNKTIONSKATHETER
CATHÉTER MULTIFONCTION

(30) Priority: 14.07.2021 US 202163221805 P; 24.05.2022 US 202263345169 P
(43) Date of publication of application: 22.05.2024
(73) Proprietor: The University of Chicago, Chicago, IL 60637 (US)
(72) Inventor: PANIAN, Tyler, Chicago, Illinois 60637 (US); PAUL, Jonathan, Chicago, Illinois 60637 (US); AHMED, Osmanuddin, Chicago, Illinois 60637 (US)
(74) Representative: J A Kemp LLP
(86) International application number: PCT/US2022/073726
(87) International publication number: WO 2023/288268

(56) References cited:
- WO-A1-2010/009407
- CN-A- 112 120 759
- US-A- 5 855 563
- US-A- 5 925 016
- US-A- 6 090 096
- US-A1- 2012 059 285
- US-A1- 2012 059 356
- US-A1- 2012 316 436
- US-A1- 2013 317 425
- US-A1- 2014 221 996
- US-A1- 2014 277 098
- US-A1- 2018 049 761
- US-A1- 2020 046 364
- US-A1- 2020 085 453

## Description

### FIELD OF DISCLOSURE

The present disclosure is generally related to venous thromboembolic disease (VTE), and, more particularly but not by way of limitation, to catheters and methods for diagnosis and/or treatment of VTE.

### BACKGROUND

Venous thromboembolic disease (VTE) is a major cause of morbidity and mortality worldwide. VTE, in general, is an umbrella term for deep vein thrombosis (DVT) and pulmonary embolism (PE). Optimal treatment of these disease processes requires high quality diagnostic imaging, careful hemodynamic monitoring (in the case of pulmonary embolism), and devices that are designed to quickly and effectively traverse complex venous anatomy. A large proportion of intermediate- and high-risk thrombi are now managed invasively, often times with a prolonged infusion of thrombolytic agents.

Current approaches to the invasive management of VTE require the use of multiple catheters, and the optimal duration of thrombolytic infusion is often not individualized to the specific needs of the patient. The current workflow for catheter-directed management of pulmonary embolism requires multiple catheter exchanges, which increases procedural time and may correspondingly increase radiation exposure to the patient and treating team. First, a hemodynamic assessment of the cardiovascular system is performed, typically using a simple balloon-tipped fluid-filled catheter. Next, this is exchanged over a wire for a standard angiography catheter, and diagnostic pulmonary angiography is performed in order to visualize the location of the thrombus and determine optimal placement of the thrombolytic infusion catheter. This is then followed by an additional catheter exchange to place the infusion catheter, and thrombolytic medication is then infused over an extended time period with the goal of dissolving the thrombus. Importantly, current solutions generally do not permit continuous pressure monitoring during thrombolytic infusion, and the total drug dose and duration of infusion are often times arbitrary. For example, under current approaches, thrombolytics are typically infused over a period of 6-24 hours. Timing of thrombolytic infusion length is often arbitrary, and factors such as subjective improvement in symptoms, oxygen saturation by noninvasive finger plethysmography, and noninvasive blood pressure monitoring are often used to aid in this important decision. Prolonged infusion of thrombolytic medications may increase risks of both minor and major (i.e. intracranial) bleeding events for a patient.

Pulmonary embolism (PE) represents a leading cause of morbidity and mortality in the United States, with as many as 600,000 cases and 100,000 deaths attributed to it each year. The Centers for Disease Control (CDC) indicates that 1 of 4 patients with PE will die suddenly without warning, and PE is the third most common cause of cardiovascular death in the United States.

For intermediate and high-risk patients with PE, catheter-based treatments have emerged as an attractive solution. The most studied method of minimally invasive treatment for PE is termed "catheter directed thrombolysis (CDT)." CDT typically involves placing one or more small catheters directly within the thrombus and infusing a thrombolytic medication such as tissue plasminogen activator (tPA) to dissolve the thrombus over 2-24 hours. CDT is generally preferred over a peripheral intravenous bolus administration of thrombolytic in all but the highest risk patients, as a slower, lower-dose infusion correlates with lower rates of life-threatening bleeding. Despite the increasing role of CDT for PE, some in the art have voiced concerns regarding its safety and efficacy.

Although CDT has been shown to be effective, some in the art have assessed that CDT may be associated with a ~2-11% risk of major adverse events including catastrophic intracranial or intra-abdominal bleeding, with the incidence of bleeding complications correlated to the duration and dosage of thrombolytic infusion. Therefore, to further improve safety and efficacy of CDT, it may be desirable to infuse the lowest possible dose of thrombolytic over the shortest effective duration. This ideal endpoint would be the point when complete lysis occurs, such that further thrombolytic may not contribute additional therapeutic benefit. As the chronicity, refraction (i.e., resistance to thrombolytic), and extent of thrombus varies in each patient, this endpoint is likely not the same for all patients. A reduction in pulmonary artery (PA) pressure during thrombolytic administration reduces right ventricular strain and correlates with an improvement in cardiac output, and an increase in wedge pressure distal to the thrombus is a clear indicator of thrombolytic efficacy. However, current CDT devices do not allow for PA pressure monitoring during thrombolytic infusion, leaving physicians to subjectively decide when to cease thrombolytic infusion and risk unnecessary lytic delivery.

### SUMMARY

The inventors have recognized certain opportunities to address the residual risks and limitations of existing CDT devices to reduce the risk of adverse events while improving technical ease of use. Embodiments of the present catheters and methods can be configured to: (1) monitor (and/or permit monitoring of) progress of thrombus disruption by way of hemodynamic monitoring, enabling objective decisions on when to end lytic delivery, and/or (2) deliver lytic agent more-closely to (e.g., from a catheter structure that is in contact with) a thrombus, thereby maximizing the efficacy of-and potentially reducing the needed amount of-lytic agent (e.g., relative to lytic agent delivered from a narrower (conventional) CDT catheter.

The aforementioned objectives are achieved according to the invention by means of a catheter system according to claim 1. Preferred embodiments are defined in the dependent claims. Some embodiments of the present catheters can be configured to provide one or more of the following benefits and features, multi-directional lytic delivery, advanced steerability, enables pulmonary angiogram, simultaneous distal/proximal pressure measurement, adjustable infusion length, added mechanism for thrombus disruption (surface contact), suitable for use in pulmonary arteries.

The present catheters and methods, these methods however not falling under the invention as claimed, allow users to perform diagnostic pulmonary angiography, hemodynamic monitoring, and infusion of medication through a single device and eliminate at least some (e.g., all) catheter exchanges. The present catheters can incorporate the functionality of a hemodynamic monitoring catheter, a diagnostic angiography catheter, and a thrombolytic infusion catheter. The present catheters and methods can be configured or implemented to enable a user to perform a detailed hemodynamic assessment and angiography at the time of diagnosis and, if the catheter is left in place, will allow for prolonged infusion of medication into the affected vessel(s). Additionally, hemodynamic monitoring ports and/or micro electrical-mechanical monitoring (MEMS) can be included to allow for real-time, continuous assessment of pulmonary artery pressures and additional physiologic metrics at the bedside. Such features will permit providers to more accurately determine the efficacy of the therapy over time, helping the user to decide when to discontinue the thrombolytic medication.

Some embodiments of the present catheters and methods target emboli in the pulmonary artery, but may be suitable for other applications in other parts of the vasculature.

In addition, more-complete thrombus resolution (dissolution) may be improved by maximizing the exposure of thrombus to the thrombolytic drug across the entire length and three-dimensional (3D) structure of the thrombus, through improved penetration and enabling infusion catheter length adjustment to individual patient needs, to achieve more complete thrombus resolution (dissolution).

Some embodiments of the present catheters comprise: an elongated catheter body having a proximal end, a distal end, and a length extending from the proximal end and the distal end, the body defining: one or more first ports extending through a peripheral surface of the body at a position along the length that is closer to the distal end than to the proximal end; a plurality of interior lumens extending longitudinally through the catheter along at least a portion of the length. In some such embodiments, the plurality of interior lumens comprises: one or more first lumens in fluid communication with the one or more first ports; and one or more tubes each having a sidewall defining a tube lumen in fluid communication with one of the first ports, the one or more tubes configured to shift between a collapsed configuration and an expanded configuration in which the one or more tubes define a cage shape having an unconstrained maximum transverse dimension that larger than a corresponding maximum transverse dimension of the catheter body, where in the collapsed configuration, at least part of tube(s) is radially closer to the catheter body than when in the expanded configuration; where at least a portion of each of the one or more tubes includes one or more openings extending through the respective sidewall in fluid communication with the respective tube lumen.

In some configurations, the one or more tubes can include a plurality of tubes. The plurality of tubes can define one or more cage shapes each having an unconstrained maximum transverse dimension that is larger than a corresponding maximum transverse dimension of the catheter body. In the collapsed configuration, at least part of the tube(s) can be radially closer to the catheter body than when in the expanded configuration. In some aspects, the unconstrained maximum transverse dimension may be at least 150%, such as at least 300%, of the corresponding maximum transverse dimension of the catheter body. In some configurations, an unconstrained maximum longitudinal dimension is at least 300% of the corresponding maximum transverse dimension of the expandable tube(s). The one or more openings can include a plurality of the openings spaced along a longitudinal portion of a length of each tube. In some configurations, the one or more tubes comprises a shape-memory alloy, such as nitinol.

Some aspects of the present catheter can include an elongated sheath having a proximal end, a distal end, and a length extending from the proximal end and the distal end, the sheath defining a sheath lumen. In some such configurations, the sheath is configured to extend over the catheter body such that the distal end of the sheath can be moved proximally relative to the catheter body to permit the one or more tubes to move from the collapsed configuration to the expanded configuration.

Some implementations of the present methods (not claimed) comprise: inserting a distal end of one of the present catheters through a clot within a (e.g., pulmonary) blood vessel of a patient such that the second port of the catheter body is distal of the clot; and retracting a sheath relative to the catheter body such that the distal end of the sheath is proximal of the clot. In some such implementations, the method further comprises: injecting a lytic agent into the blood vessel via the one or more first lumens and the opening(s) in the one or more tubes; measuring, through the second lumen and the second port, distal pressure in the blood vessel on a distal side of the clot; and/or measuring, through the sheath lumen, proximal pressure in the blood vessel on a proximal side of the clot. In some configurations, based on either a decrease/improvement or normalization of the proximal pressure, and/or equalization of the proximal and distal pressures with pressure waveforms appearing similar to a typical pulmonary artery pressure waveform, the user may choose to alter a flow rate of the lytic agent in at least one of the opening(s) in the one or more tubes. Some methods can include displaying the proximal pressure and the distal pressure, comparing the proximal pressure and the distal pressure, displaying the change in proximal and distal pressure, and based on these comparisons, terminating infusion of the lytic agent.

In some aspects, the present catheter may include an elongated catheter body having a proximal end, a distal end, and a length extending from the proximal end and the distal end. The body can define: one or more first ports extending through a peripheral surface of the body at a position along the length that is closer to the distal end than to the proximal end, one or more second ports extending through a peripheral surface of the body at a position along the length that is closer to the distal end than to the proximal end, and a plurality of interior lumens extending longitudinally through the catheter along at least a portion of the length. In some configurations, the plurality of interior lumens may include a first lumen in fluid communication with the one or more first ports, a second lumen in fluid communication with the one or more second ports, a third lumen extending through the distal end of the body to define a third port through the distal end.

Some of the present catheters can include a balloon coupled to a peripheral surface of the body at a longitudinal position that is between the distal end and the first and second ports. In some such configurations, the plurality of interior lumens can include a fourth lumen in fluid communication with the balloon such that the balloon can be inflated via a port that is coupled to and in fluid communication with the fourth lumen. In some configurations, the body includes a steerable tip extending from the distal end of the body toward the proximal end, and the plurality of interior lumens include a fo.urth lumen extending into the steerable tip to permit a user to alter the direction of the distal end relative to at least a portion of the body proximal to the steerable tip. In some aspects, the plurality of lumens each has a circular cross section. Alternatively, the third lumen can have a circular cross section and the first and second lumens each has a non-circular cross-section. In such configurations, the third lumen may have a minimum internal transverse dimension that is larger than a minimum internal transverse dimension of either of the first and second lumens. The first lumen can have an interior diameter that is larger than an interior diameter of any of the second and third lumens. In some configurations, the one or more first ports may include a plurality of first ports spanning a first region or first longitudinal extent of at least 2 centimeters (cm). The first longitudinal extent can have a length of 3 cm to 5 cm.

In some configurations, the plurality of first ports are arranged linearly along the first longitudinal extent. In some configurations, the plurality of first ports are arranged helically around the body along the first longitudinal extent. In some aspects, the one or more second ports comprise a plurality of second ports spanning a second longitudinal extent of at least 5 cm. The second longitudinal extent can have a length of 10 cm to 15 cm. The plurality of second ports can be arranged linearly along the second longitudinal extent or arranged helically around the body along the second longitudinal extent. In some configuration, the second longitudinal extent may overlap the first longitudinal extent. In some such configurations, the entirety of the first longitudinal extent is within the second longitudinal extent. The second longitudinal extent can have a first end and a second end, and the first longitudinal extent is spaced longitudinally from each of the first and second ends of the second longitudinal extent. Some of the present catheters can include a plurality of conduits or fittings, such as Luer fittings, each in fluid communication with a respective one of the lumens.

Some of the present methods (not claimed) may include inserting a distal end of the catheter through a clot within a blood vessel (e.g. pulmonary blood vessel) of a patient such that the third port is distal of the clot, and the one or more first ports are proximal of the clot. The catheter can be inserted over a guidewire extending through the third lumen and, some methods may include removing the guidewire from the third lumen. In some aspects, the method can include measuring, through the third lumen and the third port, distal pressure in the blood vessel on a distal side of the clot, measuring, through the first lumen and the one or more first ports, proximal pressure in the blood vessel on a proximal side of the clot, or both. Some methods can include injecting contrast agent into the blood vessel through the first lumen and the one or more first ports. Some methods can include the steps of injecting a lytic agent into the blood vessel via the second lumen and the one or more second ports. Some such methods can include measuring, through the first lumen and the one or more first ports, proximal pressure in the blood vessel while injecting the lytic agent, measuring, through the third lumen and the third port, distal pressure in the blood vessel while injecting the lytic agent, or both.

The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any embodiment of the present apparatuses, kits, and methods, the term "substantially" may be substituted with "within [a percentage] of" what is specified, where the percentage includes 0.1, 1, 5, and/or 10 percent.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, an apparatus or kit that "comprises," "has," "includes" or "contains" one or more elements possesses those one or more elements, but is not limited to possessing only those elements. Likewise, a method that "comprises," "has," "includes" or "contains" one or more steps possesses those one or more steps, but is not limited to possessing only those one or more steps.

In general usage, a 'thrombus' is a clot that forms inside a blood vessel, and an 'embolus' is a portion of a thrombus that breaks free and lodges itself at a point in the downstream vasculature. The disclosed invention interacts with both thrombi and emboli in the same way, so the terms are used here interchangeably. Unless specifically indicated otherwise, all references to 'thrombus' or 'embolus' (and thrombi, emboli, and embolism) apply to all thrombus and embolus related structures.

Throughout this disclosure, the terms 'proximal' and 'distal' are referenced to the catheter system of the subject invention. That is the catheter handle and user controls are on the proximal end and the portion that enters the target embolus is the distal end. Further, an apparatus, device or system that is configured in a certain way is configured in at least that way, but it can also be configured in other ways than those specifically described.

Any embodiment of any of the present apparatuses and methods can consist of or consist essentially of - rather than comprise/include/contain/have - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb.

Some details associated with the aspects of the present disclosure are described above, and others are described below. Other implementations, advantages, and features of the present disclosure will become apparent after review of the entire application, including the Brief Description of the Drawings, Detailed Description, and the Claims.

### BRIEF DESCRIPTION OF THE DRAWINGS

The following drawings illustrate by way of example and not limitation. For the sake of brevity and clarity, every feature of a given structure is not always labeled in every figure in which that structure appears. Identical labels or reference numbers do not necessarily indicate an identical structure. Rather, the same reference number may be used to indicate a similar feature or a feature with similar functionality, as may non-identical reference numbers. Dimensioned figures are drawn to scale (unless otherwise noted), meaning the sizes of the depicted elements are accurate relative to each other for at least the embodiment depicted in the figures.
FIG. 1 depicts a schematic view of an example of a catheter of the present thrombolytic catheter systems.
FIGs. 2A-2D depict various cross-sectional views of examples of catheters of the present thrombolytic catheter systems.
FIGs. 3A-3C depict various examples of components that can be utilized with the catheters of the present thrombolytic catheter systems.
FIG. 4A depicts a schematic side view of another example of a catheter of the present thrombolytic catheter systems in a first state.
FIG. 4B depicts a schematic side view of the catheter of FIG. 4A in a second state.
FIGs. 4C and 4D are cross-sectional views of the catheter of FIG. 4A taken along line A-A and B-B, respectively.
FIG. 5A depicts a schematic side view of an example of a catheter of the present thrombolytic catheter systems in an expanded state.
FIG. 5B depicts an illustrative view of an example of a catheter during a thrombolysis operation.
FIG. 6A depicts a schematic perspective view of another example of a catheter of the present thrombolytic catheter systems.
FIG. 6B depicts a schematic side view of the catheter of FIG. 6A coupled to a handle assembly.
FIGs. 7A-7E depict perspective views of the catheter of FIG. 6A in first though fifth configurations.
FIGs. 8A and 8B are cross-sectional views of an example of a catheter of the present thrombolytic catheter systems.
FIG. 9A is a cross-sectional view of another example of a catheter of the present thrombolytic catheter systems.
FIG. 9B is a perspective view of the catheter of FIG. 9A.
FIG. 10 depicts an illustrative view of an example of a catheter during a thrombolysis operation of a pulmonary artery.

### DETAILED DESCRIPTION

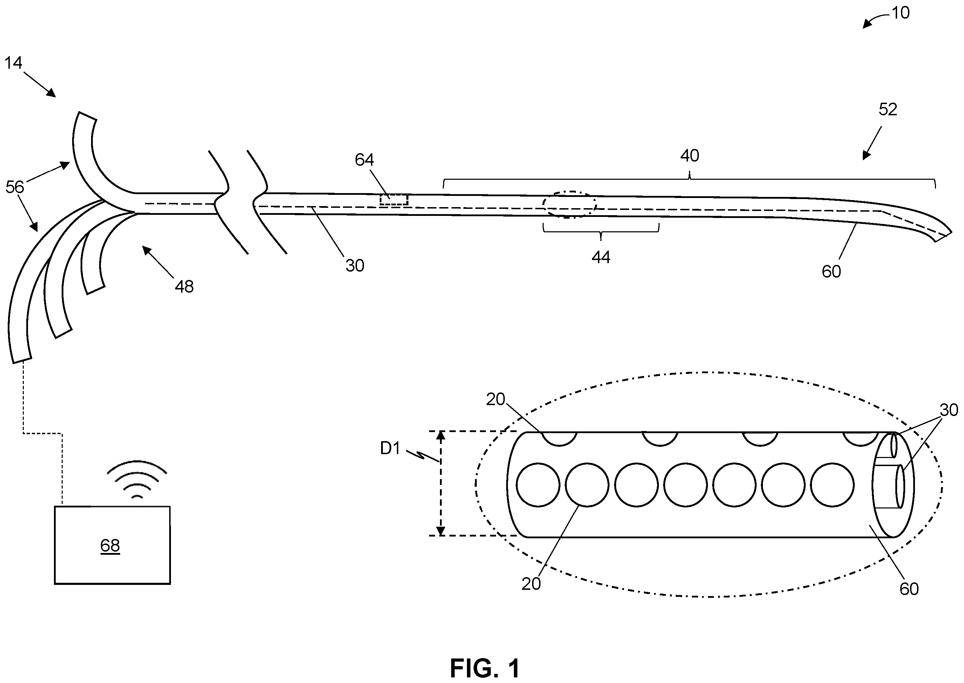

Referring now to the drawings, and more particularly to FIG. 1, shown therein and designated by the reference numeral 10 is one configuration of a catheter system. System 10 includes various components, as described herein, that are configured to facilitate thrombolysis. In the configuration depicted in FIG. 1, system 10 includes a catheter 14 having an elongated catheter body 18 configured to be disposed within the human body, such as within a vein or an artery. Catheter body 18 can include a polymer, such as PTFE, Pebax, PEEK, a metal, hard plastic braid or coil or other strong, flexible, or biocompatible materials known in the art. Catheter body 18 defines a plurality of ports 20 and a plurality of lumens 30 extending longitudinally through the body to enable fluid communication between catheter 14 and a blood stream, while the catheter is disposed within a blood vessel, to minimize procedure time and volume of infused thrombolytic agent as compared to conventional catheters. For example, catheter 14 can include a thrombolytic agent dispersion region 40 associated with one or more ports configured to supply a thrombolytic agent to the blood vessel, a contrast agent injection region 44 configured to supply a contrast agent to the blood vessel, or both. In some configurations, region 40 and region 44 can overlap one another.

Catheter body 18 extends between a proximal end 48 and a distal end 52 and defines the plurality of lumens 30 at positions between the proximal and distal ends. As shown in FIG. 1, lumens 30 can, but need not, extend along an entirety of a length defined between the proximal and distal ends. For example, one lumen (e.g., 30) can extend from proximal end 48 to contrast agent injection region 44, another lumen (e.g., 30) can extend from the proximal end to thrombolytic agent dispersion region 40, yet another lumen (e.g., 30) can extend from the proximal end to distal end 52, or combination thereof. The length of body 18 is sufficient to allow distal end 52 to extend to a embolus disposed anywhere within the human vasculature while proximal end 48 remains outside of the human body.

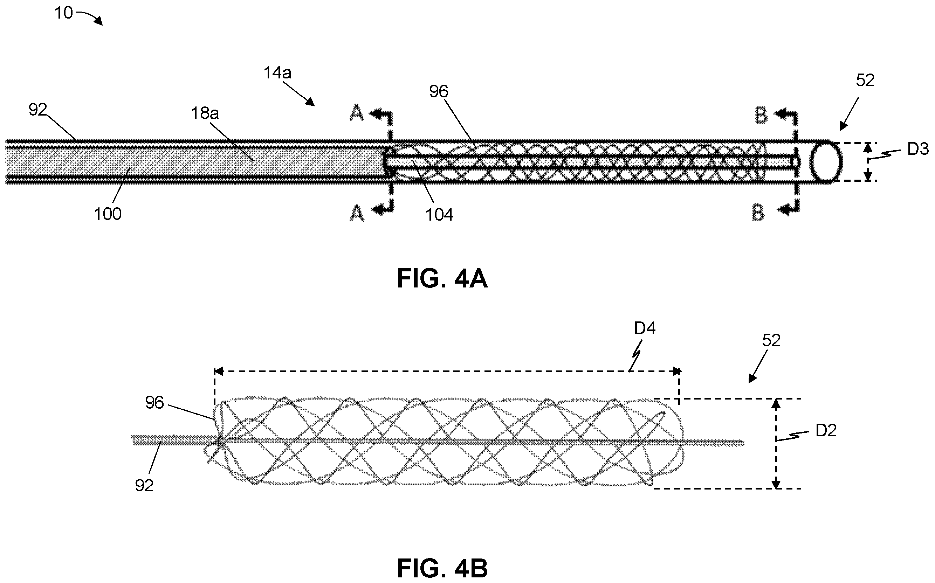

In some configurations, the contrast-injection region 44 can include ports 20 (e.g., all ports associated with a contrast-injection lumen) spaced along a 3-5 cm length of body 18 and may be spaced 8 - 12 cm from distal end 52 of catheter 14. In some configurations, thrombolytic agent dispersion region 40 can include ports 20 (e.g., all ports associated with a thrombolytic agent lumen) spaced along a 15 - 22 cm length of body 18 and may be positioned at or near distal end 52 of catheter. Ports 20 can be arranged linearly along body 18 or can be arranged in other suitable patterns. In some configurations, body 18 can include a maximum transverse dimension D1 that is less than or equal to 3 mm (9 French), such as 8 French or 7 French. Alternatively, maximum transverse dimension D1 can be larger, such as, for example, 10 French, 18 French, or greater. In some configurations, system 10 can include a sleeve that is moveable (e.g., slidable) relative to body. For example, as described in more detail with reference to FIGs. 4A-4D below, sleeve can move relative to body to block or expose one or more ports 20 associated with thrombolytic agent dispersion region 40, contrast agent injection region 44, or both.

In some configurations, each of thrombolytic agent dispersion region 40 and contrast agent injection region 44 are positioned closer to distal end 52 than proximal end 48 so that the region can inject an agent near or at the embolus. For example, in the depicted configuration, proximal end 48 diverges or branches off to form a plurality of conduits 56. Each conduit 56 can be associated with (e.g., in fluid communication with) a respective lumen of the plurality of lumens 30 and can receive an agent or other material to be dispersed in the blood vessel. Conduits 56 may include a fitting or other interface (e.g., Luer lock, Luer slip connection, twist-to-connect couplings, small bore connectors, Tuohy-Borst connector, or other known connector) that enables connection of the conduit and associated lumen(s) 30 with other components (e.g., external syringe, pump, transducer, sensor, or the like).

As shown in the enlarged cut-out of body 18 depicted in FIG. 1, the body defines one or more ports 20 extending through a peripheral surface 60 of the body. Ports 20 can be positioned along the length of body 18 at a location that is closer to distal end 52 than to proximal end 48. In some configurations, ports 20 include apertures connecting a respective lumen (e.g., 30) to an exterior of body 18. For example, a series of first ports (e.g., 20) can be positioned within thrombolytic agent dispersion region 40 and be in fluid communication with a first lumen (e.g., 30) to deliver a thrombolytic agent from proximal end 48 (e.g., via a first conduit 56) to the thrombolytic agent dispersion region. Additionally, or alternatively, a series of second ports (e.g., 20) can be positioned within contrast agent injection region 44 and be in fluid communication with a second lumen (e.g., 30) to deliver a contrast agent from proximal end 48 (e.g., via a second conduit 56) to the contrast agent injection region.

In some configurations, system 10 can include one or more sensors 64, one or more controllers 68, or both. As shown in FIG. 1, sensor 64 can be coupled to (e.g., integrated with) body 18. Sensor(s) 64 can be coupled to body 18 at any position along its length and, in some configurations, can be disposed at a location that is closer to distal end 52 than proximal end 48. In some configurations, sensor 64 is configured to detect or measure clinically relevant data. For example, sensor 64 may include or correspond to a micro electrical-mechanical sensing (MEMS) device that is configured to measure pressure with high sensitivity, an integrated oxygen sensor, a flow sensor, pressure sensor, blood oxygen saturation sensor, pH sensor, hemoglobin sensor, chemical sensors, strain gauges, blood flow sensor, or combination thereof. Sensor 64 can be associated with one or more ports 20, one or more lumens 30, or both, to detect and transmit information from the sensor to a device (e.g., controller 68) that will communicate this information to an operator. In an illustrative configuration, sensor 64 can be disposed within body 18 (e.g., in a sidewall) and wiring can extend through the body through a designated lumen (e.g., 30) and through conduits 56. In some configurations, one or more wires is/are coupled to sensor 64 and extend though the body of catheter 14 can connect to an external console or controller 68. In some configurations, sensor can include electrical sensors, in which the transducing elements are located in situ on catheter 14, and wires extend back to proximal end 48 to communicate power and data, such as the TiSense pressure sensor by Millar corporation. In some configurations, sensors 64 may include optical sensors, such as the RJ50 by RJ Enterprises, in which Fabry-Perot cavity or other optical sensors are placed in situ on catheter 14, each with an optical fiber running back to proximal end 48. Controller 68 or sensor can be configured to model hemodynamics in real-time and be configured for high fidelity sensing (including pressure, oxygen, pH and flow) including shock states (such as cardiogenic, distributive, hemorrhagic, or obstructive), intra-operative monitoring, or trauma.

Controller 68 can be in wired or wireless communication with one or more other components of system 10, such as sensor 64, a fluid source (e.g., pump), pressure transducer, monitor, medical machinery (e.g., imaging systems, vital monitor, or the like), control system, or other electrical component typically utilized during a thrombolysis procedure. Controller 68 may include a processor coupled to a memory (e.g., a computer-readable storage device). In some configurations, controller 68 may include one or more application(s) that access processor and/or memory to perform one or more operations of system 10, as described herein. Processor may include or correspond to a microcontroller/microprocessor, a central processing unit (CPU), a field-programmable gate array (FPGA) device, an application-specific integrated circuits (ASIC), another hardware device, a firmware device, or any combination thereof. Memory , such as a non-transitory computer-readable storage medium, may include volatile memory devices (e.g., random access memory (RAM) devices), nonvolatile memory devices (e.g., read only memory (ROM) devices, programmable read-only memory, and flash memory), or both. Memory may be configured to store instructions, one or more thresholds, one or more data sets, or combination thereof. In some configurations, instructions (e.g., control logic) may be configured to, when executed by the one or more processors, cause the processor(s) to perform one or more operations (e.g., determining pressures at various positions along catheter 14, transmitting alerts based on measured parameters, displaying parameters or notifications on a display, actuating a fluid source, or the like). The one or more thresholds and one or more data sets may be configured to cause the processor(s) to generate control signals to perform the operations. As described herein, system 10 can include real-time monitoring during thrombolysis that is capable of shortening therapy time and utilizing a lower effective dose of thrombolytic drug to improve patient safety, as compared to traditional catheter systems.

In some configurations, system 10 can include a user-interface (UI) configured to display useful parameters such as the proximal and distal pressure waveforms, the pressure averages, the pressure and flow rate of infusion, the total time of infusion, the total volume of thrombolytic infused, as described herein. The UI may be in communication controller 68 and may be configured to be utilized with instructions (e.g., an algorithm) to determine when the proximal and distal waveforms are sufficiently alike to assume complete thrombolysis has occurred. The UI may have an algorithm that monitors thrombolytic agent infusion pressure and flowrate, and signals to the user when it is likely that the embolus is fully lysed. The controller 68 can initiate one or more alerts, visible via the UI, when flowrate is high, possibly indicating infusion in an area outside the embolus. In configurations in which expandable tubes (e.g., 96) are arranged such that individual tubes only infuse certain zones along the length of catheter 14, the UI may indicate where the embolus is likely still present, based in flowrates in different zones. The UI may display a graphic image of the embolus to show its estimated present shape and size. In some configurations, the UI may alert the user if a threshold in lytic quantity or time has been exceeded, or if a threshold in contrast quantity or time has been exceeded. The UI may alert the user if the proximal pressure sensor (e.g., 64) is not showing a valid pulmonary artery waveform. The UI may also monitor and give threshold alerts relating to typical signs of thrombolytic efficacy, such as decreased pulmonary artery (proximal sensor) pressure and increased pulmonary capillary wedge (distal sensor) pressure. The UI may record data for future download / upload. The UI may utilize learning algorithms that determine optical thrombolytic dosages for future patients based on successful outcomes with past patients. The UI may upload its parametric data to a shared database, which can form the basis of machine learning for improved thrombolysis parameter settings.

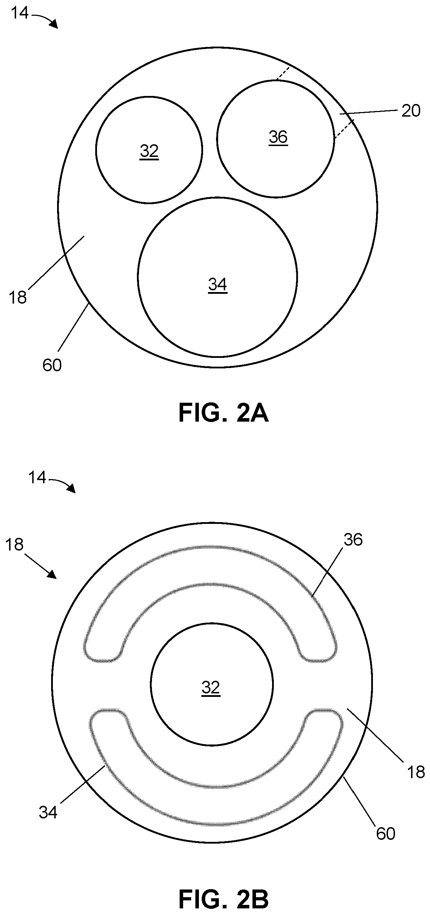

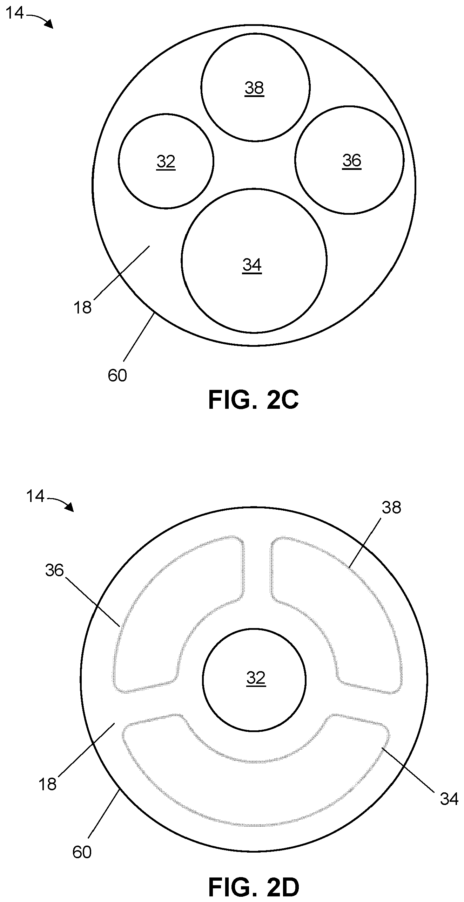

Referring now to FIGs. 2A-2D, cross-sectional view of various configurations of body 18 are shown and described in greater detail. For example, FIGs. 2A and 2B depict three-lumen configurations of body 18 having a first lumen 32, a second lumen 34, and a third lumen 36 and FIGs. 2C and 2B depict three-lumen configurations of the body having the first lumen, the second lumen, the third lumen, and a fourth lumen 38. Each of first lumen 32, second lumen 34, third lumen 36, and fourth lumen 38 can have a variety of geometries and are not limited to the shapes and sizes shown in FIGs. 2A-2D.

In some configurations, first lumen 32 can be configured to operate as a guidewire lumen. In such configurations, first lumen 32 can extend along an entire length of catheter 14, from proximal end 48 to a distal tip. First lumen 32 can be sized to receive a guide wire and can have a maximum transverse dimension that is greater than or equal to 0.035 inches (in), such as 0.04 in, 0.05 in, or more. In some configurations, a minimum internal transverse dimension of lumen 32 is greater than that of second, third, or fourth lumens 34, 36, 38. First lumen 32 may be configured to measure a distal pressure, such as a pressure on a distal side of a thrombus. For example, first lumen 32 can be in fluid communication with an opening at distal end 52 (e.g., at the distal tip) and connected to a pressure transducer to measure the pressure of a blood vessel at the distal end of body 18. In some configurations, such as those shown in FIGs. 2B and 2D, first lumen 32 is centered within body 18. In other configurations, first lumen 32 may have a longitudinal axis that is closer to a longitudinal axis (e.g., the centerline) of the body than to the peripheral surface 60.

In some configurations, second lumen 34 can be configured to operate as contrast-injection lumen. In some configurations, such as when second lumen 34 is circular, the second lumen may have a maximum transverse dimension that is greater than or equal to 1.33 mm (4 French), such as 5 French, 6 French, or more. Second lumen 34 can be in fluid communication with a series of ports 20 (second ports) disposed along region 44 and configured to deliver a contrast agent within the blood stream to diagnostic quality angiographic images. For example, second lumen 34 can be in communication with a second conduit (e.g., 56) that is connected to a fluid source (e.g., pump, syringe, or other pressurized source configured to inject fluid into a blood stream) having a contrast agent configured for fluoroscopy, MRI, CT, PET, or other medical imaging modalities. Additionally, or alternatively, second lumen 34 can be configured to measure a proximal pressure, such as a pressure on a proximal side of the thrombus. For example, second lumen 34 can be connected to a pressure transducer (e.g., at the second conduit) to measure a pressure at the second ports (e.g., 20). This connection may be made in parallel with the fluid source (e.g., contrast injecting device) using a T-piece, or optionally with a valve or manifold arrangement to isolate the fluid source and the pressure sensor.

Using first and second lumens 32, 34, catheter 14 can detect a pressure at two different points in a blood stream during operation. Sensing pressure at longitudinally-spaced positions (proximal and distal to a thrombus) enables real-time analysis of blood flow restoration within a vessel via analysis of pressure waveforms both upstream and downstream of a thrombus. For example, within and distal to a thrombus, a pressure waveform (e.g., pulmonary artery pressure waveform) will be blunted due to the lack of pulsatile flow through the thrombus. As thrombus resolves, such as during the delivery of a lytic-agent, the distal pressure waveform will generally become more typical. Thus, system 10 or an operator thereof, can approximate normalization of blood flow in the vessel within or distal to a thrombus to determine when thrombolysis is complete. This capability will therefore allow a lower effective dose of thrombolytic drug to be administered, thereby minimizing the risk of bleeding to the patient that may otherwise increase with administration of more lytic agent than may be necessary to restore normal blood flow.

In some configurations, third lumen 36 can be configured to operate as a lytic-agent dispersion lumen. In some configurations, such as when third lumen 36 is circular, the third lumen may have a maximum transverse dimension that is greater than or equal to 1.0 mm (3 French), such as 5 French, 6 French, or more. Third lumen 36 can be in fluid communication with a series of ports 20 (third ports) disposed along region 40 and configured to continuously deliver a thrombolytic agent within the blood stream treat a thrombus. For example, third lumen 36 can be in communication with a third conduit (e.g., 56) that is connected to a fluid source (e.g., pump, syringe, or other pressurized source configured to inject fluid into a blood stream) having a lytic-agent. Third lumen 36 is distinct from first and second lumens 32, 34 and injection of a fluid (e.g., lytic-agent) in third lumen 36 can be performed without interfering with the functions of the first and second lumens (e.g., pressure detection).

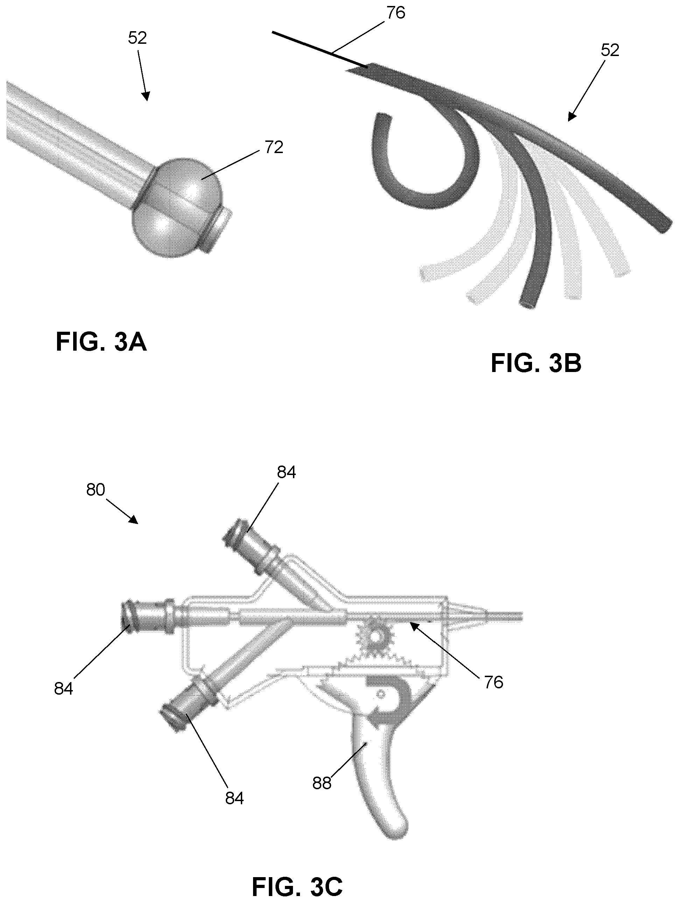

Referring now to FIGs. 2C and 2D, body 18 can include fourth lumen 38. Fourth lumen 38 can be utilized for various functions, such as those described above with reference to first, second, or third lumens 32, 34, 36 for redundancy. In some configurations, fourth lumen 38 can be utilized with a balloon (e.g., as shown in FIG. 3A) disposed at or near distal end 52. In such configurations, fourth lumen 38 can be in fluid communication with the balloon to inflate and deflate the balloon to navigate the catheter through human vasculature, such as through the heart and into the pulmonary vasculature. In other configurations, fourth lumen 38 can be utilized with a steerable tip (e.g., such that distal tip can be manipulated over a 180 degree range) to facilitate easier navigation and wire selection of different branches of the pulmonary arterial tree. In such a configuration, fourth lumen 38 can be used to control the steerable tip (e.g., as shown in FIG. 3B). In some configurations in which catheter 14 includes a sleeve, fourth lumen 38 can be utilized to move the sleeve relative to body 18, such as via a wire extending through the fourth lumen 38.

Although described as being utilized for treatment of pulmonary embolism, catheter 14 can also be employed in other applications, for example intra-vascular thrombolytic therapy, and utilize real-time hemodynamic monitoring and self-expandable cage/basket lytic delivery, as described herein. For example, catheter 14 can facilitate treatment of both arterial and venous thrombosis, including but not limited to deep venous thrombosis, arterial lower extremity thrombosis, renal vein thrombosis, mesenteric venous thrombosis, and IVC-filter related thrombosis. In some applications, catheter 14 can be modified based on the treatment area. As an illustrative example, in the deep venous and mesenteric/visceral venous space, catheter 14 can have a larger profile to provide great surface contact of lytic agent with thrombus along with greater length of infusion adjustability. Some such illustrates, may also include a larger mechanism for mechanical thrombus removal along with a distal caval protection iteration to avoid distal emboli. For example, in some configurations, the catheter can be substantially 10 French to help facilitate contrast injection or can be up to substantially 18 French for thrombectomy. In other application, catheter can be of lower profile to adjust for smaller vessel caliber and lesser need for surface contact with the vessel wall. A distal embolic protection mechanism may also be similarly employed.

It should be understood that each of first lumen 32, second lumen 34, third lumen 36, and fourth lumen 38 may be utilized for a different function that that described above. In some configurations, this can be alternative to or in addition to the described function. For example, the described lumens can be utilized to aspirate blood samples for testing, such as during an index procedure or while the catheter is being used for drug infusion and/or hemodynamic monitoring.

Referring now to FIGs. 3A-3C, catheter 14 may include or be operable with one or more other components to increase the functionality of the catheter. For example, FIG. 3A depicts an inflatable balloon 72 disposed on distal end 52 of body 18 that is configured to expand and contract to navigate blood vessels and FIG. 3B depicts the distal end having a steerable tip that is configured to rotate relative to a more proximal portion of the body. The steerable tip can include wiring, such as a steering cable 76 to manipulate the distal tip. Balloon 72 can be positioned between distal end 52 and ports 20. In some configurations, balloon 72 and the steerable tip may be controlled at proximal end 48, such as via a lumen (e.g., 38) or one or more conduits (e.g., 56).

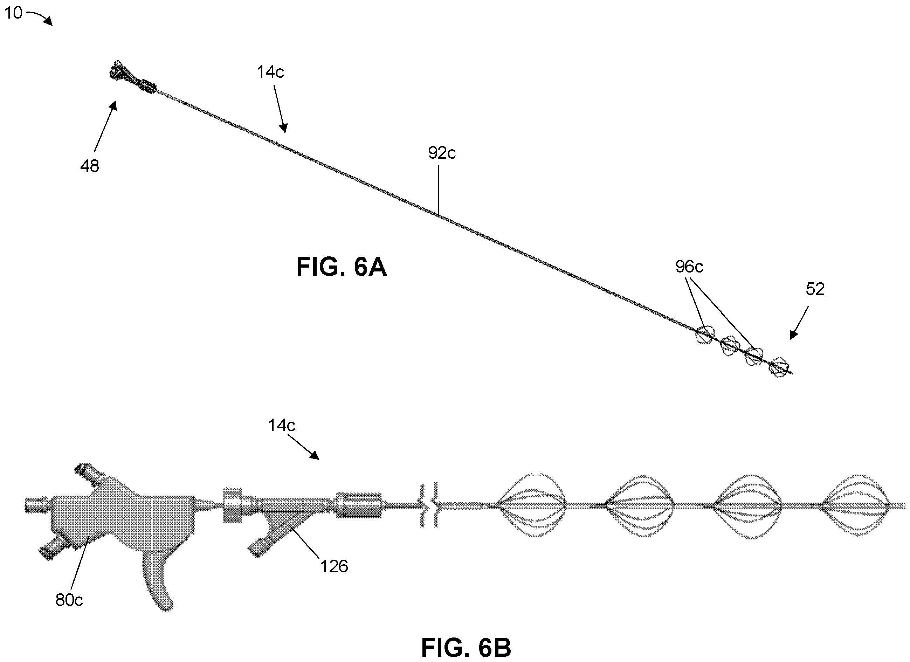

In some configurations, system 10 may include a handle assembly 80, as shown in FIG. 3C, that is disposed at proximal end 48 to facilitate the control of catheter 14 by an operator. As shown, handle assembly 80 includes a plurality of fittings 84 configured to engage with a respective conduit 56 or lumen 30. The depicted handle assembly 80 includes three fittings 84. In some configurations, a first fitting (e.g., 84) is configured to provide or facilitate Lytic infusion, a second fitting (e.g., 84) is configured to provide or facilitate use with a guide wire, and a third fitting (e.g., 84) is configured to provide or facilitate use with a balloon (e.g., 72). In some such configurations, the second fitting may be connected to a distal pressure transducer, such as for example, via a T-piece or valve assembly to allow parallel functions . In some configurations, handle assembly 80 can include additional fittings or can be coupled to another components with additional fittings (e.g., 126 as shown in FIG. 6B) configured to provide or facilitate contrast injection, connect to a proximal pressure transducer, or both. In some configurations, handle assembly 80 includes a steering mechanism 88 that includes a handle connected to gearing such that movement of the handle moves the gearing. In such configurations, steering wire can be coupled to steering mechanism 88 to adjust a distal end 52 of body based on movement of the handle. Steering mechanism need not include gearing or a handle and can be configured to manipulate a steering wire as is understood in the art, such as via a simple pull wire, a push rod, a rotary knob, a motorized retraction system, or other systems.

Referring now to FIGs. 4A-4D, a second configuration of shown therein and designated by the reference numeral 14a is a second configuration of the present catheters of system 10. In this configuration, components that are similar (e.g., in structure and/or function) to components discussed with reference to FIGS. 1-3C are labeled with the same reference numerals and a suffix "a."

FIG. 4A depicts a distal end 52 of catheter 14a that includes a body 18a (e.g., inner sheath) disposed within an outer sheath 92. Body 18a includes one or more expandable tubes 96 that are configured to radially expand relative to body 18a. While the plural "tubes" is used throughout to reflect that multiple tube segments define the overall shape of expandable tubes 96, those multiple tube segments may in some embodiments be parts of a single, continuous tube. Outer sheath 92 is independently movable relative to body 18 selectively cover or expose portions of body 18a. For example, as shown in FIG. 4A, outer sheath 92 is disposed over expandable tubes 96 such that the tubes are in a collapsed state. As shown in FIG. 4B, outer sheath 92 can be moved relative to body 18 to reveal expandable tubes 96 such that the tubes are in an expanded state in which the tubes have an unconstrained maximum transverse dimension D2 that is larger than a corresponding maximum transverse dimension of body 18a or outer sheath 92. While expandable tubes 96 are in the collapsed state, at least a part of the tubes are radially closer to body 18a than when in the expanded state to reduce the overall diameter of catheter 14a (as compared to the expanded state) to facilitate ingress through the vasculature to an embolus. Movement of outer sheath 92 can be controlled at proximal end (e.g., 48) as described above. In some configurations, outer sheath 92 and body 18a may include fluoroscopic markings to assist navigation.

In some configurations, the unconstrained maximum transverse dimension D2 of expandable tubes 96 can be at least 150% (e.g., greater than any one, or between any two, of: 150%, 200%, 250%, 300%, 350%, 400%, 450%, and/or 500% of) the maximum transverse dimension D3 of outer sheath 92. As shown in FIG. 4B, while in the expanded state, expandable tubes 96 can have an elongated shape. For example, expandable tubes 96 can have a unconstrained maximum longitudinal dimension D4 that is greater than unconstrained maximum transverse dimension D2. In some configurations, unconstrained maximum longitudinal dimension D4 can be greater than 500% of unconstrained maximum transverse dimension D2. In other configurations, unconstrained maximum longitudinal dimension D4 may be greater than any one, or between any two, of: 125%, 150%, 200%, 250%, 300%, 350%, 400%, 450%, 500%, 550%, 600%, 650%, 700%, and/or 750% of unconstrained maximum transverse dimension D2.

Expandable tubes 96 can be formed of nitinol that is configured to exhibit hyperelastic or shape memory properties. For example, expandable tubes 96 may be formed of laser cut or extruded nitinol tubing that, in some configurations, is wound about a mandrel and heat-treated to a desired shape. In some configurations, expandable tubes 96 may include multiple materials, such as for example, a distal portion of the tubes could include nitinol and a proximal portion could include PTFE or other polymer. In some configurations, tubes 96 can be in fluid communication with a lumen of body 18a and can be configured to deliver fluid (e.g., a lytic agent) within a blood vessel. Expandable tubes 96 can be positioned nearer distal end 52 than proximal end 48 and can be configured to expand into a shape conducive to uniform, isotropic infusion of thrombolytic agent throughout the internal volume of the target embolus. Although not shown for clarity, expandable tubes 96 can include a plurality of ports, such as ports 20, disposed along its length to allow thrombolytic agent to flow into the blood vessel. The shape expandable tubes 96 can be configured to disperse fluid in multiple direction and may increase the surface area available for infusion as compared to conventional catheters. In an illustrative configuration, expandable tubes 96 may assume a helical, spiral, spherical, conical, flower, cage, or basket shape while in the expanded state.

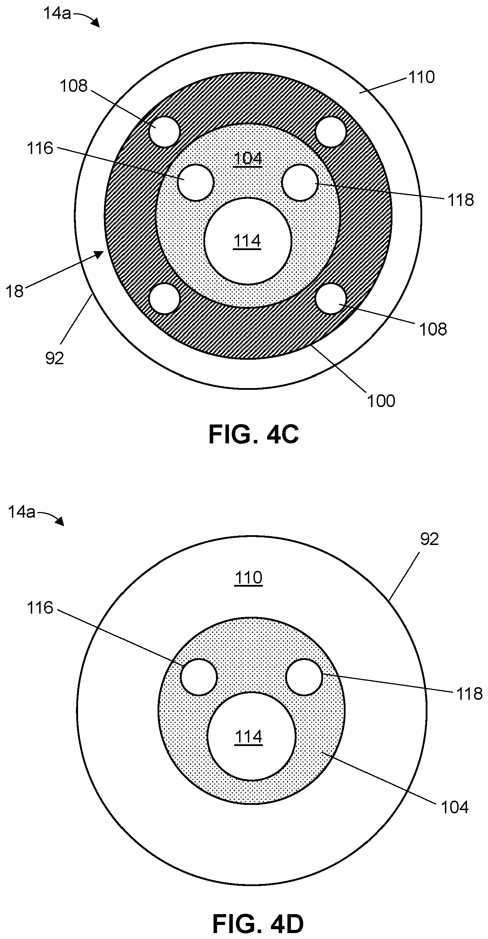

Referring now to FIGs. 4C and 4D, section views of catheter 14a are shown. As illustrated best in FIG. 4C, body 18a may include an outer body 100 and an inner body 104. In some configurations, a length of outer body 100 can be less than a length of inner body 104 such that a distal tip of the outer body is more proximal than a distal tip of the inner body. Outer body 100 may surround inner body 104 and can include a channel configured to receive the inner body. Outer body 100 and inner body can have a variety of geometries (e.g., number and size of lumens or ports) , such as circular, rectangular, hexagonal, curved, elliptical, or the like and are not limited to the shapes and sizes shown in FIGs. 4A-4D.

Outer body 104 can include a plurality of lumens 108 that are configured to be in fluid communication with expandable tubes 96. In some configurations, lumens 108 may include or correspond to lumen 36 of catheter 14. In some configurations, each lumen 108 is configured to be coupled to an end of a respective tube of the expandable tubes 96 and, in other configurations, a pair of lumens (e.g., 108) can be coupled to opposing ends of an expandable tube, or other configuration. Lumens 108 may be coupled to expandable tubes 96 in any suitable manner to allow fluid communication, such as via a heat fit, adhesive, a molding process, or any other process known in the art. Expandable tubes 96 can be disposed with lumens 108 and extend partially along the lumens or can extend all the way to proximal end 48. In some configurations, lumens 108 may fluidly connect together near proximal end 48 to be driven by a fluid sources (e.g., single thrombolytic agent infusion pump or syringe) or, in other configurations, lumens 108 may remain separated and be driven by separate sources, or by a single source through a manifold or valve arrangement. Although four lumens 108 are shown with corresponding connections to expandable tubes 96, any number of lumens and corresponding connections to expandable tubes may be employed. In an alternative embodiment, expandable tubes 96 may not be part of body 18a and may extend down the length of the body and held loosely inside outer sheath 92 and the body (e.g., inner body 104).

As shown in FIGs. 4C and 4D outer sheath defines a lumen 110 in which body 18a is disposed. An inner diameter of outer sheath 92 can be larger than the outer diameter of outer body 100 such that lumen 110 can function as described herein. In an illustrative example, outer sheath 92 can include an inner diameter of substantially 2 mm and outer body 100 can have an outer diameter of substantially 1 mm such that lumen 110 may define a 1 mm annular gap at line A-A and a larger gap at line B-B. Lumen 110 may be configured to operate a contrast injection lumen, a proximal pressure lumen, or both. For example, during thrombolysis, outer sleeve 92 will be positioned proximal of a thrombus so that extendable tubes 96 may shift to the expanded state. During operation, a pressure transducer can be connected to a proximal end of lumen 110 to measure a pressure at the distal end of the lumen at a point proximal to the thrombus. In some configurations, lumens 110 may include or correspond to lumen 34 of catheter 14.

Inner body 104 includes a plurality of lumens extending along at least a portion of the length of the inner body, from a proximal end (e.g., 48) to a distal end (e.g., 52). In the depicted configurations, inner body 104 includes a first lumen 114, a second lumen 116, and a third lumen 118. Each of first, second, and third lumen 114, 116, 118 can include or correspond to lumens 32, 34, 36, or 38, described above. For example, first lumen 114 can include a guidewire lumen that is sized to receive a guidewire (e.g., 0.018" or 0.035" diameter guidewire) and can be configured to measure a distal pressure, such as a pressure on a distal side of a thrombus. First lumen 114 can be in fluid communication with an opening at distal end 52 (e.g., at the distal tip) of inner body 104 and connected to a pressure transducer to measure the pressure of a blood vessel at the distal end of body 18a. In some configurations, lumens 114 may include or correspond to lumen 32 of catheter 14. In an illustrative example, second lumen 116 may be configured as a steering lumen and may be configured to receive a steerable wire to facilitate movement of distal end 52 of body 18a. Additionally, or alternatively, third lumen 118 may be configured as a balloon lumen and can be configured to inflate and deflate a balloon coupled to distal end 52 of catheter 14a. In some configurations, lumens 116, 118 may include or correspond to lumen 38 of catheter 14. It should be understood that catheter 14a (e.g., body 18a) can include more or less lumens than are depicted.

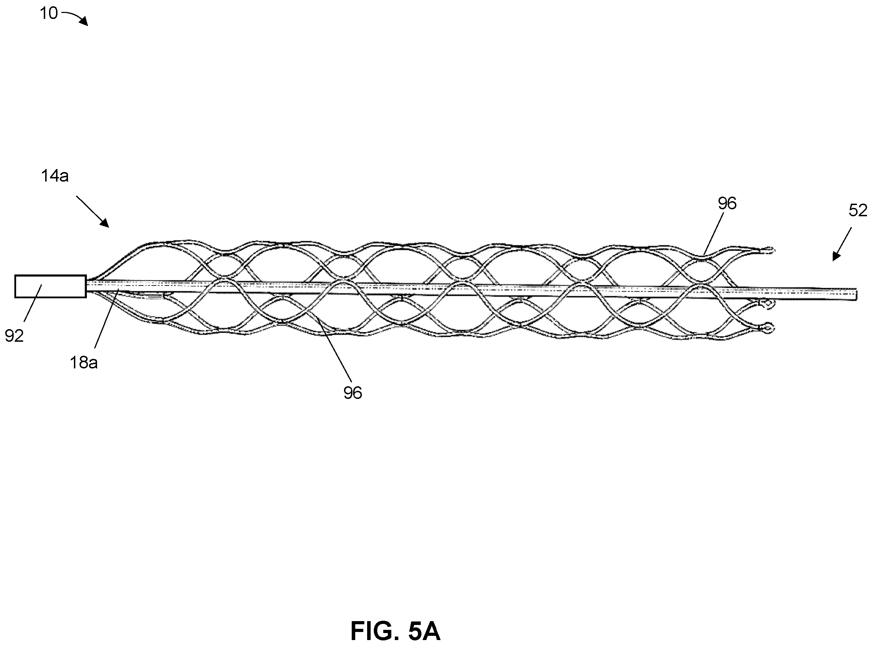

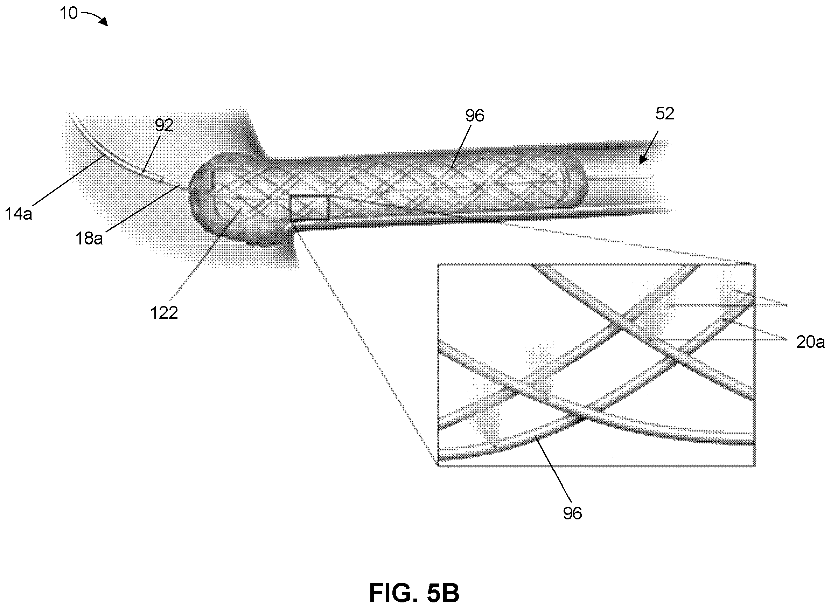

As shown in FIG. 5A and 5B a configuration of catheter 14a and a method of operating the catheter are shown. Catheter 14a can have expandable tubes 96 that include nitinol tubes. As shown in FIG. 5A expandable tubes 96 can have a first end that extend from outer sheath 92 and a second end near distal end 52. In some configurations, the second end may include or correspond to a return point in which a portion (e.g., midpoint) of expandable tubes 96 changes direction. In some such configurations, an inlet an outlet of expandable tubes 96 may be positioned at first end, or nearer first end than second end. FIG. 5B illustrates a method of operating catheter 14a for treating a pulmonary embolism in the pulmonary artery, however, a skilled person would understand the catheter can be utilized for other . In use, catheter 14a may be inserted at a suitable venous access point, for example the femoral, jugular, brachial, or subclavian vein. In some embodiments, catheter 14a may be navigated to an embolus 122 via a balloon, guidewire, steering wire, or combination thereof, as understood in the art. During navigation, outer sheath 92 may cover body 18a and compress expandable tubes 96 until reaching embolus 122. Proximal end (e.g., conduit 56) of lumen 110 or 114 may be fluidly coupled to a fluid source containing imaging contrast agent to inject the contract agent either by manual or mechanical means. In some configurations, an operator can inject contrast through lumen 110 during ingress to locate embolus 122. The contrast agent may be appropriate for fluoroscopy, MRI, CT, PET, or other medical imaging modalities.

When distal end 52 of the catheter 14a reaches embolus 122, the operator may advance it through the embolus until the distal tip is just distal to the embolus, as shown in FIG. 5. In some implementations, this position may be confirmed by further contrast injection through the lumen 110 or by connecting a catheter pressure transducer to the proximal end of lumen 110 (e.g., at the conduit), or both via a parallel connection with the contrast injecting device, using a T-piece, or manifold arrangement. In configurations in which lumen 110 is configured to detect a pressure, when a distal most tip of outer sheath 92 is inside the pulmonary artery but proximal to embolus 122, a typical pulmonary artery pressure waveform can be detected. In some such configurations, the waveform can be displayed on a monitor or some other indication of a normal pressure reading may be initiated. When the distal most tip of outer sheath 92 is distal to embolus 122, a typical pulmonary capillary wedge pressure waveform can be detected and, in some configurations, displayed or otherwise indicated.

With both sheaths (e.g., 92, 18a) of catheter 14a fully through embolus 122, the operator can expose inner body 104, releasing expandable tubes 96, and begin the infusion process, as shown in FIG. 5. Using controls at the proximal end of the catheter, such as a handle assembly (e.g., 80) the operator can retract outer sheath 92 while inner body 104 remains in place. These proximal controls may consist of a simple pull wire, a push rod. a rotary knob, a motorized retraction system, or other systems obvious to those in the art. Expandable tubes 96 can expand radially from body 18a to the expanded state and are fluidly coupled to lumens (e.g., 108) in the body that extend back to at least one conduit on proximal end of catheter 14a, which connects to a fluid source for infusing thrombolytic agent through ports 20a in the expandable tubes 96. The infusion device may be a hand syringe, or an infusion pump as are well known in the art. Expandable tubes 96 can be perforated at numerous points along their length with ports (e.g., holes or skives) facing different directions normal to the tubes central axis (defined when the expandable tube is in a linear configuration). The ports may be drilled holes, cut skives, chemically etched or the expandable tubes 96 can be manufactured as a mesh or porous material. In some configurations, ports (e.g., 20) can be sized and shaped to create desirable spray patterns when combined with known pressures within the capability of the fluid source. For example, it may be desirable to place micronozzles or atomizers on each port to shape the spray pattern.

As described above, the distal tips of both outer sheath 92 and body 18a distal tips may provide separate lumens that extend back to the catheter proximal end and terminate in fluid connections to commercial pressure transducers, such as the IP-BD-300 by Becton Dickinson. These two measurement points may be used to compare blood pressure between the proximal and distal sides of embolus 122. For example, when outer sheath 92 is retracted to a point where a normal pulmonary artery pressure waveform is detected, the operator understand that the outer sheath retraction is sufficient and should be stopped. This can ensure that expandable tubes 96 is only exposed inside embolus 122 and does not extend more proximal to non-occluded portions of the artery. In this way, and others, catheter 14a can minimize an area in which a thrombolytic agent is delivered and minimize the amount of thrombolytic that is infused to areas outside embolus 122, thus providing minimal risk to the patient.

The distal tip of body 18a (e.g., inner body 104) remains distal to embolus 122 during retraction of outer sheath 92 and can detect a typical capillary wedge pressure. During thrombolytic infusion, the operator may monitor both pressure waveforms to ensure proper position is maintained. As the thrombolytic agent gradually dissolves embolus 122, the 'distal pressure' waveform (capillary wedge pressure) will change until it is nearly identical to the 'proximal pressure' waveform (normal pulmonary artery pressure waveform). At this point, the operator may discontinue thrombolytic agent infusion and conclude the procedure. In this way and others, system 10 can perform thrombolysis with minimal infusion quantity and time under anesthesia and fluoroscopy.

Additionally, or alternatively, fluoroscopic markings can assist with positioning catheter 14a. For example, outer sheath 92 may have a marking at its distal tip, and body 18a (e.g., outer body 100, inner body 104) may be provided with graduated ruled fluoroscopic rings to facilitate measurement of the length of the exposed part of the body, and to indicate the axial length of the portion of expandable tubes 96 that is in the expanded state. Further, contrast agent may be injected into either the lumen 110 or lumen 114, allowing the operator to position inner body 104 at the most proximal point that still shows no blockage, and outer sheath 92 at the most distal point that shows blockage. Positioning the two sheaths as close as possible to the ends of embolus 122 can reduce infusion of thrombolytic agent upstream or downstream of the embolus.

As a specific, non-limiting example, system 10 can include a flowrate measurement via thermodilution. To illustrate, catheter 14a can inject a bolus of cold saline or other fluid into lumen 110 and a thermistor or other type of temperature sensor may be disposed at the distal end of the catheter. System 10 can measure the time between cold saline injection and the sensing of a temperature drop by the thermistor. From this, flowrate and possibly flow volume can be estimated using algorithms known in the art.

Throughout the infusion period, the operator may use the pressure measurements or contrast injection to reposition the two sheaths (e.g., 92, 18a) in response to changes in the embolus size and shape. The operator may adjust the position of either sheaths tip to ensure expandable tubes 96 are infusing agent only inside the embolus volume, and not in the upstream or downstream spaces. As the operator can move outer sheath 92 relative to body 18a to compress portions of expandable tubes 96 and block the ports associated with the compresses portion of the tubes. In this way, the operator can tailor the axial length of the infusion zone to the length of embolus 122 in real time.

In some configurations, the present catheter (e.g., 14, 14a, 14b) can be utilized for thrombectomy in addition to or alternative to thrombolysis. For example, expandable tubes 96 can be configured to be positioned near a thrombus in the expanded state and move to the collapsed state to engage the thrombus. The catheter may then be retracted to remove the engaged thrombus. In some configurations, the large diameter (e.g., unconstrained maximum transverse dimension) of the expandable tubes 96 can enable removable of the thrombus via direct interaction with the expandable tubes. In configurations, in which body (e.g., 18, 18a) is moved over a guidewire, the catheter can be removed with thrombus trapped within the interwoven cage formed by expandable tubes 96. As most thrombectomy procedures fail to remove the full extent of thrombus via manual or mechanical techniques, significant thrombus often remains, increasing the risk of long term complications, such as heart failure, pulmonary hypertension, or chronic thromboembolic disease. Therefore, after initial attempts at thrombectomy, the present catheter (e.g., 14, 14a, 14b, 14c, 14d) may then be placed back into the region of interest, and a thrombolytic medication (such as tissue plasminogen activator or "tPA") may be infused through the methods described herein. Multiple thrombolytic outlet holes (e.g., 20a) traverse the length of the catheter, allowing thrombus in the vicinity or downstream of the catheter to be subject to pharmacologic thrombolysis. Thus, the present catheter can be enable a combination of mechanical disruption and removal of clot (i.e. thrombectomy) with catheter-directed thrombolysis (i.e. lysis) using a single device. In this way and other, the present catheters can allow users to more completely treat patients with extensive thromboembolic disease.

Referring now to FIGs. 6A and 6B, shown therein and designated by the reference numeral 14c is a third configuration of the present catheters of system 10. In this configuration, components that are similar (e.g., in structure and/or function) to components discussed with reference to FIGS. 1-5 are labeled with the same reference numerals and a suffix "b." Catheter 14b is similar to catheter 14a except expandable tubes 96 are configured to form a plurality of individually spaced cages, rather than a single elongated cage. As shown, while expandable tubes 96 are in the expanded state, one or more spherical cages are formed extending radially from body 18c. As shown in FIG. 6B, catheter 14c can be coupled to a handle assembly 80c and a fitting assembly 126 that can provide additional ports or conduits to connect to the lumens of the catheter.

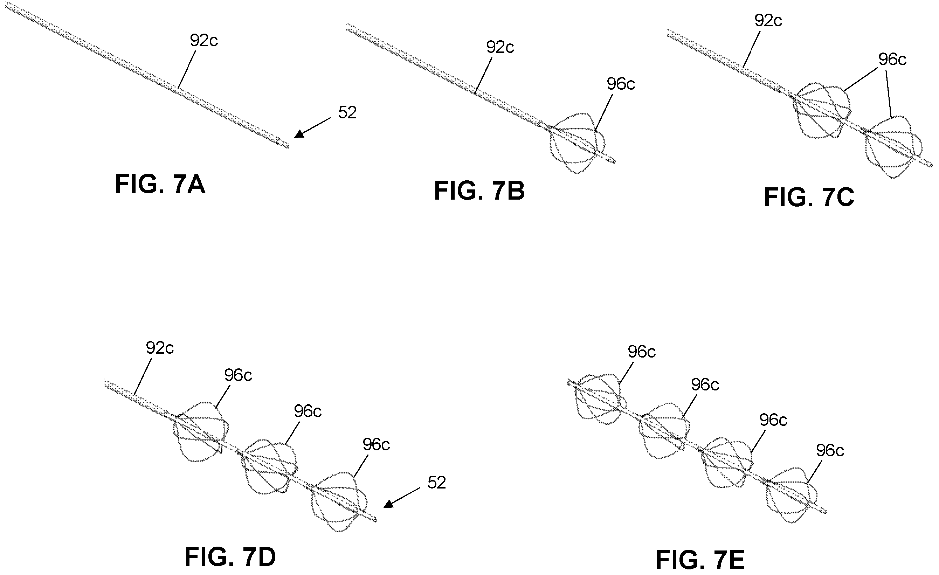

Referring now to FIGs. 7A-7E, an outer sheath 92c is shown being retracted relative to body 18c to reveal a series of expandable tubes 96c. To start outer sheath 92c completely covers body 18c (FIG. 7A) and the outer sheath can be retracted while the body remains in place to uncover a first set of expandable tubes 96c (FIG. 7B) that can include a single tube or a plurality of tubes cooperating to radially expand upon being uncovered. Outer sheath 92c can be further retracted to reveal a second set of expandable tubes 96c (FIG. 7C), a third set of expandable tubes 96c (FIG. 7D), and a fourth set of expandable tubes 96c (FIG. 7E). As outer sheath 92c is retracted or advanced, more sets of expandable tubes or cages are released or compressed, allowing the operator to adjust the effective length of a lytic infusion region to match the length of the embolus. In some configurations, each cage can be operated independently of one another. To illustrate, an operator could deploy all four expandable tube cages in the embolus and begin infusion. If each cage is attached to a separate fluid source (e.g., lytic infusion source), each source can monitor pressure and flowrate of infusion into its respective expandable tube cage 96c. If one of the cages 96c shows a drop in pressure or a rise in flowrate, this may indicate that that cage has lysed its portion of the embolus. In this case, the operator, or the infusion source acting automatically, could stop infusion to that cage only while maintaining infusion in the other cages. In some configurations, an operator can reposition catheter 14c based on these or other parameters. Thus, monitoring flowrate and pressure at the infusion source may be another useful means to detect the status of thrombolysis in different parts of the embolus, alone or in conjunction with contrast injection and pressure waveform analysis, and adapt infusion to the changing embolus shape. Although FIGs. 7A-7E depict four spherical expandable tube cages (e.g., 96c), more or less cages can be implemented in catheter 14c, such as a single cage, or two, three or five cages.

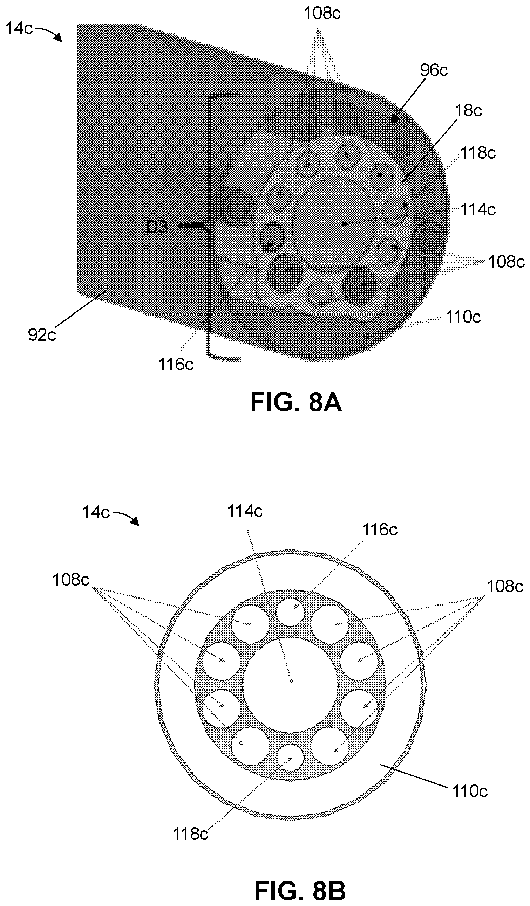

FIGs. 8A and 8B shows an example of a sectional view of catheter 14c. In this configurations, catheter 14c includes outer sheath 92c and body 18c having a plurality of lumens. As shown, body 18c can include a plurality of lumens 108c configured to be connected to expandable tubes 96c and deliver thrombolytic agent into the blood stream, a lumen 114c configured to receive and travel over a guidewire, a lumen 116c configured as a steering lumen and may be configured to receive a steerable wire to facilitate movement of distal end 52 of body 18c, a lumen 118c configured as a balloon lumen and configured to inflate and deflate a balloon coupled to distal end 52 of catheter 14a, or combination thereof. Outer sheath 92c includes a lumen 110c configured to operate a contrast injection lumen, a proximal pressure lumen, or both. In some configurations, expandable tubes 96c depicted as outside body 18c can connect to lumens 108c further upstream or proximal (left in FIG. 8A) and the expandable tubes shown within lumens 108c can exit body 18c further downstream or distal (right in FIG. 8A), such as for example, via one or more ports (e.g., 20) in body 18c.

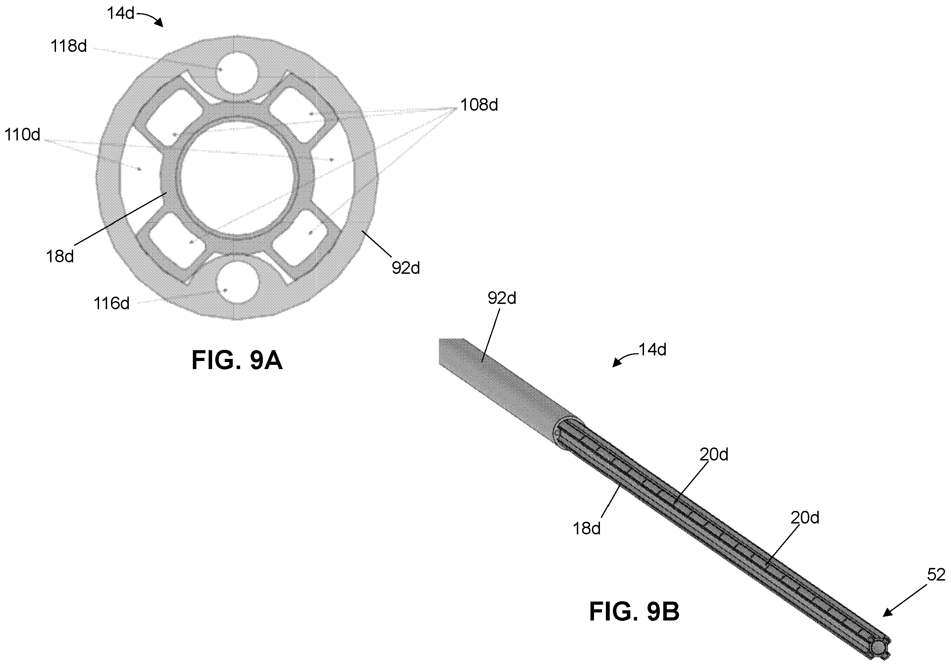

Referring now to FIGs. 9A and 9B, shown therein and designated by the reference numeral 14d is a fourth configuration of the present catheters of system 10. In this configuration, components that are similar (e.g., in structure and/or function) to components discussed with reference to FIGS. 1-8B are labeled with the same reference numerals and a suffix "d." Catheter 14d includes an outer sleeve 92d having a lumen 118d configured as a balloon lumen and configured to inflate and deflate a balloon coupled to distal end of catheter 14d, a lumen 116d configured as a steering lumen and may be configured to receive a steerable wire to facilitate movement of the distal end of body 18d, and a lumen 110d in which body 18d is disposed and is configured to operate a contrast injection lumen, a proximal pressure lumen, or both. Body 18d can includes a plurality of lumens 108d configured to be connected to expandable tubes and deliver thrombolytic agent into the blood stream. As shown in FIG. 9B, lumens 108d can include a plurality of ports 20d extending through a surface of body 18d to enable fluid communication between lumens 108d and a blood vessel. In some configurations, expandable tubes (e.g., 96c) can be coupled to lumens 108d and extend through ports 20d to move between a compressed and expanded state based on a relative position of outer sheath 92d.

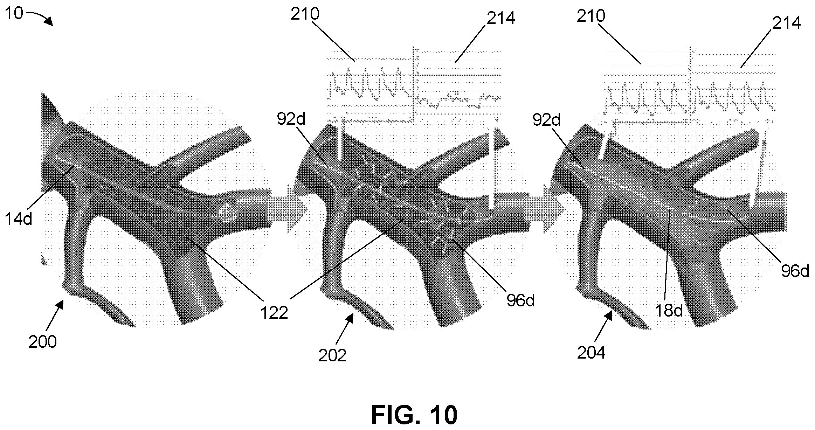

FIG. 10 illustrates an exemplary method by which the present catheters (e.g., 14, 14a, 14b, 14c, 14d) may be used to treat an embolus. For FIG. 10, components that are similar (e.g., in structure and/or function) to components discussed above are labeled with the suffix "d" but and can include or correspond to any of the above described components (e.g., "a," "b," "c,"). The first illustration (left figure) of an artery at a first time 200 shows a cutaway closeup of a segment of the pulmonary artery with an embolus 122. The catheter 14d may be inserted at a suitable venous access point, for example the femoral, jugular, brachial, or subclavian vein. In some embodiments catheter 14d may provide a balloon for flow guidance to the pulmonary artery as shown. In other embodiments, catheter 14d may provide a lumen configured to track over a guidewire as is known in the art. In further embodiments, catheter 14d may provide a steering wire to assist tracking to the target embolus. The outer sheath 92d may cover body 18d and compress the expandable tubes 96d during ingress and navigation to embolus 122. Either outer sheath 92d or body 18d may be provided with fluoroscopic markings to assist navigation. Further, the outer sheath 92d inner diameter may be relatively large compared to that of the body 18d , and the proximal end of the outer sheath may be fluidly coupled to an inlet port to allow injection of imaging contrast agent, either by manual or mechanical means. The contrast agent inlet conduit may connect to an external syringe or pump via a luer fitting or other means known in the art. The implanting physician may inject contrast through outer sheath 92d during ingress to locate embolus 122. In an exemplary embodiment, outer sheath 92d may have an inner diameter of approximately 2 mm and body 18d may have an outer diameter of approximately 1 mm. The contrast agent may be appropriate for fluoroscopy, MRI, CT, PET, or other medical imaging modalities.

When the distal end of catheter 14d reaches embolus 122, the operator advances it through the embolus until the distal tip is just distal to the embolus, as shown at time 200, in which the left side of the figure is proximal and the right distal. This position may be confirmed by further contrast injection through outer sheath 92d. Alternatively, position may be confirmed by connecting a catheter pressure transducer to the proximal end at the port which fluidly connects to the outer sheath. This connection may be made in parallel with the contrast injecting device, using a T-piece, or optionally with a valve or manifold arrangement to isolate the injector and the pressure sensor. When outer sheath 92d tip is inside the pulmonary artery but proximal to embolus 122, a typical pulmonary artery pressure waveform 210 can be visible on the transducer output display, shown at time 202. When outer sheath 92d tip is distal to the embolus, a typical pulmonary capillary wedge pressure waveform 210 can be seen.

Body 18d may contain a relatively large lumen that is opens at the distal tip of the catheter, and extends back to another luer port on the proximal end. In guidewire directed embodiments, this lumen may be used to hold the guidewire, for example 0.018" or 0.035" diameter guidewire, and the proximal port may contain a hemostatic fitting for the guidewire such as a Tuohy-Borst connector.

With both Sheaths of the catheter fully through embolus 122, the operator can expose body 18d and begin infusion, as shown at time 202. Using controls at the proximal end of catheter 14d, the operator retracts outer sheath 92d while holding body 18d in place. These proximal controls may consist of a simple pull wire, a push rod. a rotary knob, a motorized retraction system, or other systems obvious to those in the art. At time 202, outer sheath 92d is pulled proximal (towards left in the frame), releasing the compressed expandable tubes 96d and allowing it to expand radially from body 18d. The expandable tubes 96d inlets are fluidly coupled to lumens in body 18d that extend back to at least one port on the catheter's proximal end, which connects to a device for infusing thrombolytic agent. The infusion device may be a hand syringe, or an infusion pump as are well known in the art. The expandable tubes 96d are perforated at numerous points along their length with holes or skives facing different directions normal to the tube's elongate axis. The perforations may be drilled holes, cut skives, or the expandable tubes 96d could be manufactured as a mesh or porous material. Holes or skives may be drilled or cut mechanically, with lasers, or with chemical etching processes.

As described above, both outer sheath 92d and body 18d's distal tips may provide separate lumens that extend back to the catheter proximal end and terminate in fluid connections to commercial pressure transducers, such as the IP-BD-300 by Becton Dickinson. Once the guidewire is removed from body 18d, these two measurement points may be used to compare blood pressure between the proximal and distal sides of embolus 122, as shown at times 202 and 204. When outer sheath 92d is retracted to a point where a normal pulmonary artery pressure waveform displays as 'proximal pressure', the operator knows outer sheath 92d retraction is sufficient and should be stopped. This ensures that expandable tubes 96d are only exposed inside the volume of the embolus, and minimizes the amount of thrombolytic that is infused to areas outside the embolus, providing maximum therapeutic benefit and minimal risk to the patient.

In addition to using pressure waveforms to ascertain optimal Sheath retraction, the catheter may be provided with fluoroscopic markings, which, when combined with contrast injection, can assist in optimizing the position of both other sheath 92d and body 18d. For example, outer sheath 92d may have a marking at its distal end, and body 18d may be provided with graduated ruled rings to facilitate measurement of the length of the exposed part of the body, and to indicate the axial length of the expanded portion of expandable tubes 96d. Further, contrast agent may be injected into either the outer sheath 92d or body 18d, allowing the operator to position the body at the most proximal point that still shows no blockage, and outer sheath 92d at the most distal point that shows blockage. Positioning the two sheaths as close as possible to the ends of the embolus minimize infusion of thrombolytic agent upstream or downstream of embolus 122.

While outer sheath 92d displays a pulmonary artery pressure waveform as 'proximal pressure', body 18d distal tip has remained distal to embolus 122 and displays a typical capillary wedge pressure waveform 214 as 'distal pressure', as shown at time 202. During thrombolytic infusion, the operator may monitor both waveforms to ensure proper position is maintained. As the thrombolytic agent gradually dissolves embolus 122, the 'distal pressure' waveform will change until it is nearly identical to the 'proximal pressure' waveform, as shown at time 204. At this point, the operator may discontinue thrombolytic agent infusion and conclude the procedure, minimizing patient risks associated with infusion quantity and time under anesthesia and fluoroscopy. Likewise, multiple infusion points throughout the embolus volume ensure optimal diffusion of thrombolytic agent and minimize the time of thrombolysis.

Throughout the infusion period, the operator may use the pressure measurements or contrast injection to reposition catheter 14d in response to changes in the embolus size and shape. The operator may adjust the position of either outer sheath 92d or body 18d to ensure the expandable tubes 96d is infusing agent only inside the embolus volume, and not in the upstream or downstream spaces. As the operator retracts body 18d back into outer sheath 92d, part of the expandable tubes 96d is again compressed and infusion ports are blocked by outer sheath 92d. In this way, the operator can tailor the axial length of the infusion zone to the length of the embolus in real time. When the procedure is complete, the operator fully retracts body 18d into outer sheath 92d and removes the catheter 14d.

The above specification and examples provide a complete description of the structure and use of exemplary embodiments. Although certain embodiments have been described above with a certain degree of particularity, or with reference to one or more individual embodiments, those skilled in the art could make numerous alterations to the disclosed embodiments without departing from the scope of this invention, which is defined by the appended claims. As such, the various illustrative embodiments of the present devices are not intended to be limited to the particular forms disclosed. Rather, they include all modifications and alternatives falling within the scope of the claims, and embodiments other than the one shown may include some or all of the features of the depicted embodiment. For example, components may be combined as a unitary structure, and/or connections may be substituted. Further, where appropriate, aspects of any of the examples described above may be combined with aspects of any of the other examples described to form further examples having comparable or different properties and addressing the same or different problems. Similarly, it will be understood that the benefits and advantages described above may relate to one embodiment or may relate to several embodiments.

## Claims

1. A catheter system (10) comprising a catheter (14), the catheter (14) comprising:
an outer sheath (92);
an elongated catheter body (18a) having a proximal end, a distal end, and a length extending from the proximal end to the distal end, wherein the body (18a) is disposed within a lumen (110) of the outer sheath (92) and wherein the outer sheath (92) is independently moveable relative to the body (18a) to selectively cover or expose portions of the body (18a),
the body (18a) defining:
an outer body (100), wherein an inner diameter of the outer sheath (92) is larger than an outer diameter of the outer body (100);
an inner body (104) including a plurality of lumens extending along at least a portion of the length of the inner body (104), wherein a length of outer body (100) is less than a length of inner body (104) such that a distal tip of the outer body (100) is more proximal than a distal tip of the inner body (104);
one or more expandable tubes (96) including a plurality of ports (20) and configured to radially expand relative to the body (18a), the one or more expandable tubes (96) being configured to deliver fluid within a blood vessel via the plurality of ports (20);
where the plurality of lumens of the inner body (104) comprises:
a first lumen (114) in fluid communication with an opening at a distal end (52) of the inner body (104);
a second lumen (116); and
a third lumen (118);
the catheter (14) further comprising:
a proximal pressure sensor (64) connected to a proximal end of the lumen (110) of the outer sheath (92) to measure a proximal pressure of the blood vessel at the distal end of the lumen (110) of the outer sheath (92) when the outer sheath (92) is positioned proximal to a thrombus in the blood vessel during use; and
a distal pressure sensor (64) connected to the first lumen (114) to measure a distal pressure of the blood vessel at the distal end (52) of the body (18a) when the distal end (52) is positioned distal to the thrombus in the blood vessel during use.

2. The catheter system of claim 1, wherein the catheter system (10) further comprises:
a controller (68) connected to the proximal pressure sensor (64) and the distal pressure sensor (64) and configured to perform operations including one or more of:
displaying on a user interface display the proximal pressure and/or the distal pressure;
displaying on a user interface display a proximal pressure waveform associated with the proximal pressure and/or a distal pressure waveform associated with the distal pressure;
displaying on a user interface display a change in the proximal pressure and/or the distal pressure;
alerting a user if the proximal pressure sensor is not showing a valid pulmonary artery waveform;
comparing the distal pressure and the proximal pressure;
comparing the distal pressure waveform and the proximal pressure waveform; and
provide threshold alerts in response to one or both of a) a decrease in a proximal pressure measured by the proximal pressure sensor (64), and b) an increase in a distal pressure measured by the distal pressure sensor (64).

3. The catheter system of claim 2, wherein the catheter system (10) further comprises the user interface display in communication with the controller (68).

4. The catheter system (10) of claim 2 or 3, wherein the controller (68) is configured to compare the distal pressure waveform and the proximal pressure waveform, and determine when the distal pressure waveform and the proximal pressure waveform are sufficiently alike to assume complete thrombolysis of the thrombus has occurred.

5. The catheter system (10) of any of claims 2 to 4, wherein the controller (68) is configured to compare the distal pressure and the proximal pressure and, based on the comparison of the distal pressure waveform and the proximal pressure waveform, actuate a fluid source to terminate infusion of the fluid via the ports (20).

6. The catheter system (10) of any preceding claim, where each of the plurality of lumens of the inner body (104) has a circular cross section.

7. The catheter system (10) of claim 6, where the first lumen (114) has an interior diameter that is larger than an interior diameter of any of the second and third lumens (116, 118).

8. The catheter system (10) of any preceding claim, wherein:
when the outer sheath (92) is disposed over the one or more expandable tubes (96), the expandable tubes (96) are in a collapsed state; and
when the outer sheath (92) is moved proximally relative to body (18a) to reveal the expandable tubes (96), the expandable tubes (96) expand to an expanded state in which the expandable tubes (96) have an unconstrained maximum transverse dimension (D2) that is larger than a maximum transverse dimension of the body (18a) or the outer sheath (92).

9. The catheter system (10) of claim 8, wherein, while the expandable tubes (96) are in the collapsed state, at least a part of the expandable tubes (96) is radially closer to the body (18a) than when the expandable tubes (96) are in the expanded state.

10. The catheter system (10) of claim 8 or 9, wherein the one or more expandable tubes (96) assume shape in the expanded state selected from the group consisting of: helical, spiral, spherical, conical, flower, cage, or basket shape.

11. The catheter system (10) of any of claims 8 to 10, where the unconstrained maximum transverse dimension (D2) is at least 150%, optionally at least 300%, of the maximum transverse dimension of the body (18a).

12. The catheter system (10) of any preceding claim, where the one or more expandable tubes (96) comprise a shape-memory alloy.

13. The catheter system (10) of claim 12, where the shape-memory alloy comprises nitinol.

14. The catheter system (10) of claim 12 or 13, wherein the one or more expandable tubes (96) further comprise a polymer.

15. The catheter system (10) of any preceding claim, wherein the one or more expandable tubes (96) are configured to disperse fluid in multiple directions.

## Patentansprüche

1. Kathetersystem (10) mit einem Katheter (14), wobei der Katheter (14) umfasst:
eine Außenhülle (92);
einen länglichen Katheterkörper (18a) mit einem proximalen Ende, einem distalen Ende und einer Länge, die sich vom proximalen Ende zum distalen Ende erstreckt, wobei der Körper (18a) innerhalb eines Lumens (110) der Außenhülle (92) angeordnet ist und wobei die Außenhülle (92) relativ zum Körper (18a) unabhängig bewegbar ist, um Abschnitte des Körpers (18a) optional abzudecken oder freizulegen,
wobei der Körper (18a) Folgendes definiert:
einen Außenkörper (100), wobei ein Innendurchmesser der Außenhülle (92) größer ist als ein Außendurchmesser des Außenkörpers (100);
einen Innenkörper (104) der eine Vielzahl von Lumen enthält, die sich entlang mindestens eines Teils der Länge des Innenkörpers (104) erstrecken, wobei der Außenkörper (100) eine geringere Länge hat als der Innenkörper (104), sodass eine distale Spitze des Außenkörpers (100) proximaler liegt als eine distale Spitze des Innenkörpers (104);
eine oder mehrere expandierbare Röhren (96), die eine Vielzahl von Öffnungen (20) enthalten und so konfiguriert sind, dass sie sich relativ zum Körper (18a) radial ausdehnen, wobei die eine oder mehreren expandierbaren Röhren (96) so konfiguriert sind, dass sie über die Vielzahl von Öffnungen (20) Flüssigkeit in ein Blutgefäß abgeben;
wobei die Vielzahl der Lumen des Innenkörpers (104) umfassen:
ein erstes Lumen (114), das in Kommunikation mit einer Öffnung an einem distalen Ende (52) des Innenkörpers (104) steht;
ein zweites Lumen (116); und
ein drittes Lumen (118);
wobei der Katheter (14) ferner umfasst:
einen proximalen Drucksensor (64), der mit einem proximalen Ende des Lumens (110) der Außenhülle (92) verbunden ist, um einen proximalen Druck des Blutgefäßes am distalen Ende des Lumens (110) der Außenhülle (92), wenn die Außenhülle (92) während des Gebrauchs proximal zu einem Thrombus im Blutgefäß positioniert ist; sowie
einen distalen Drucksensor (64), der mit dem ersten Lumen (114) verbunden ist, um einen distalen Druck des Blutgefäßes am distalen Ende (52) des Körpers (18a) zu messen, wenn das distale Ende (52) während des Gebrauchs distal zum Thrombus im Blutgefäß positioniert ist.

2. Kathetersystem nach Anspruch 1, wobei das Kathetersystem (10) ferner umfasst:
eine Steuerung (68), die mit dem proximalen Drucksensor (64) und dem distalen Drucksensor (64) verbunden ist und so konfiguriert ist, dass sie Vorgänge durchführt, die eines oder mehrere der Folgenden enthalten:
Anzeigen des proximalen Drucks und/oder des distalen Drucks auf einer Benutzeroberflächenanzeige;
Anzeigen einer dem proximalen Druck zugeordneten proximalen Druckwellenform und/oder einer dem distalen Druck zugeordneten distalen Druckwellenform auf einer Benutzeroberflächenanzeige;
Anzeigen einer Änderung des proximalen Drucks und/oder des distalen Drucks auf einer Benutzeroberflächenanzeige;
Warnen eines Benutzers, falls der proximale Drucksensor keine gültige Pulmonalarterien-Wellenform anzeigt;
Vergleichen des distalen Drucks und des proximalen Drucks;
Vergleichen der distalen Druckwellenform und der proximalen Druckwellenform; und
Ausgeben von Schwellenwertwarnungen als Reaktion auf eines oder beide der Folgenden: a) einen Abfall des vom proximalen Drucksensor (64) gemessenen proximalen Drucks und b) einen Anstieg des vom distalen Drucksensor (64) gemessenen distalen Drucks.

3. Kathetersystem nach Anspruch 2, wobei das Kathetersystem (10) ferner die mit der Steuerung (68) in Kommunikation stehende Benutzeroberflächenanzeige umfasst.

4. Kathetersystem (10) nach Anspruch 2 oder 3, wobei die Steuerung (68) für Folgendes konfiguriert ist: Vergleichen der distalen Druckwellenform und der proximalen Druckwellenform; und Bestimmen, wann die distale Druckwellenform und die proximale Druckwellenform ausreichend ähnlich sind, um anzunehmen, dass eine vollständige Thrombolyse des Thrombus stattgefunden hat.

5. Kathetersystem (10) nach einem der Ansprüche 2 bis 4, wobei die Steuerung (68) so konfiguriert ist, dass sie den distalen Druck und den proximalen Druck vergleicht und, basierend auf dem Vergleich der distalen Druckwellenform und der proximalen Druckwellenform eine Fluidquelle betätigt, um die Infusion des Fluids über die Anschlüsse (20) zu beenden.

6. Kathetersystem (10) nach einem der vorhergehenden Ansprüche, wobei jede der zahlreichen Lumen des Innenkörpers (104) einen kreisförmigen Querschnitt aufweist.

7. Kathetersystem (10) nach Anspruch 6, wobei das erste Lumen (114) einen Innendurchmesser aufweist, der größer ist als der Innendurchmesser des zweiten oder des dritten Lumens (116, 118).

8. Kathetersystem (10) nach einem vorhergehenden Anspruch, wobei:
wenn die Außenhülle (92) über der einen oder den mehreren expandierbaren Röhren (96) angeordnet ist, sich die expandierbaren Röhren (96) in einem zusammengefalteten Zustand befinden; und
wenn die Außenhülle (92) relativ zum Körper (18a) proximal verschoben wird, um die expandierbaren Röhren (96) freizulegen, sich die expandierbaren Röhren (96) in einen expandierten Zustand ausdehnen, in dem die expandierbaren Röhren (96) eine maximale Querabmessung (D2) im unbelasteten Zustand aufweisen, die größer ist als eine maximale Querabmessung des Körpers (18a) oder der Außenhülle (92).

9. Kathetersystem (10) nach Anspruch 8, wobei, während sich die expandierbaren Röhren (96) im zusammengefalteten Zustand befinden, mindestens ein Teil der expandierbaren Röhren (96) radial näher am Körper (18a) liegt als im expandierten Zustand der expandierbaren Röhren (96).

10. Kathetersystem (10) nach Anspruch 8 oder 9, wobei die eine oder mehreren expandierbaren Röhren (96) im expandierten Zustand eine Form annehmen, die aus der Gruppe ausgewählt ist, bestehend aus: einer schraubenförmigen, spiralförmigen, kugelförmigen, konischen, blumenförmigen, käfigförmigen oder korbförmigen Form.

11. Kathetersystem (10) nach einem der Ansprüche 8 bis 10, wobei die maximale Querabmessung im unbelasteten Zustand (D2) mindestens 150 %, optional mindestens 300 %, der maximalen Querabmessung des Körpers (18a) beträgt.

12. Kathetersystem (10) nach einem vorhergehenden Anspruch, wobei die eine oder mehrere expandierbaren Röhren (96) eine Formgedächtnislegierung umfassen.

13. Kathetersystem (10) nach Anspruch 12, wobei die Formgedächtnislegierung Nitinol umfasst.

14. Kathetersystem (10) nach Anspruch 12 oder 13, wobei die eine oder mehreren expandierbaren Röhren (96) ferner ein Polymer umfassen.

15. Kathetersystem (10) nach einem vorhergehenden Anspruch, wobei die eine oder mehreren expandierbaren Röhren (96) so konfiguriert sind, dass sie Flüssigkeit in mehrere Richtungen verteilen.

## Revendications

1. Système de cathéter (10) comprenant un cathéter (14), ledit cathéter (14) comprenant :
une gaine externe (92),
un corps de cathéter allongé (18a) comportant une extrémité proximale, une extrémité distale et une longueur s'étendant de l'extrémité proximale à l'extrémité distale, le corps (18a) étant disposé à l'intérieur d'une lumière (110) de la gaine externe (92) et la gaine externe (92) étant indépendamment mobile par rapport au corps (18a) pour recouvrir ou exposer sélectivement des parties du corps (18a),
le corps (18a) définissant :
un corps externe (100), dans lequel le diamètre intérieur de la gaine externe (92) est supérieur au diamètre extérieur du corps externe (100),
un corps interne (104) comportant une pluralité de lumières s'étendant le long d'au moins une partie de la longueur du corps interne (104), la longueur du corps externe (100) étant inférieure à la longueur du corps interne (104), si bien qu'une extrémité distale du corps externe (100) est plus proximale qu'une extrémité distale du corps interne (104),
au moins un tube expansible (96) comprenant une pluralité d'orifices (20) et conçu pour subir une expansion radiale par rapport au corps (18a), le ou les tubes expansibles (96) étant conçus pour apporter, par le biais de la pluralité d'orifices (20), du fluide dans un vaisseau sanguin ;
la pluralité de lumières du corps interne (104) comprenant :
une première lumière (114) en communication fluidique avec une ouverture située à une extrémité distale (52) du corps interne (104),
une deuxième lumière (116), et
une troisième lumière (118) ;
le cathéter (14) comprenant en outre :
un capteur de pression proximal (64) relié à une extrémité proximale de la lumière (110) de la gaine externe (92) pour mesurer une pression proximale du vaisseau sanguin à l'extrémité distale de la lumière (110) de la gaine externe (92) lorsque la gaine externe (92) est située à proximité d'un thrombus dans le vaisseau sanguin pendant l'utilisation, et
un capteur de pression distal (64) relié à la première lumière (114) pour mesurer une pression distale du vaisseau sanguin à l'extrémité distale (52) du corps (18a) lorsque l'extrémité distale (52) est située distalement par rapport au thrombus dans le vaisseau sanguin pendant l'utilisation.

2. Système de cathéter selon la revendication **1,** le système de cathéter (10) comprenant en outre :
un dispositif de commande (68) relié au capteur de pression proximal (64) et au capteur de pression distal (64) et conçu pour exécuter des opérations comprenant au moins l'une des suivantes :
l'affichage, sur un écran d'interface utilisateur, de la pression proximale et/ou de la pression distale,
l'affichage, sur un écran d'interface utilisateur, d'une forme d'onde de pression proximale associée à la pression proximale et/ou une forme d'onde de pression distale associée à la pression distale,
l'affichage, sur un écran d'interface utilisateur, d'une variation de la pression proximale et/ou de la pression distale,
l'alerte d'un utilisateur, si le capteur de pression proximale ne montre pas une forme d'onde d'artère pulmonaire valide,
la comparaison de la pression distale et de la pression proximale,
la comparaison de la forme d'onde de pression distale et de la forme d'onde de pression proximale, et
la fourniture d'alertes de seuil en réaction à : a) une diminution de la pression proximale mesurée par le capteur de pression proximale (64), et/ou b) une augmentation de la pression distale mesurée par le capteur de pression distale (64).

3. Système de cathéter selon la revendication 2, le système de cathéter (10) comprenant en outre l'écran d'interface utilisateur en communication avec le dispositif de commande (68).

4. Système de cathéter (10) selon la revendication 2 ou 3, dans lequel le dispositif de commande (68) est conçu pour comparer la forme d'onde de pression distale et la forme d'onde de pression proximale, et pour déterminer à quel moment la forme d'onde de pression distale et la forme d'onde de pression proximale sont suffisamment semblables pour supposer qu'une thrombolyse complète du thrombus s'est produite.

5. Système de cathéter (10) selon l'une quelconque des revendications 2 à 4, dans lequel le dispositif de commande (68) est conçu pour comparer la pression distale et la pression proximale et, en fonction de la comparaison de la forme d'onde de pression distale et de la forme d'onde de pression proximale, actionner une source de fluide pour mettre fin à la perfusion du fluide par les orifices (20).

6. Système de cathéter (10) selon l'une quelconque des revendications précédentes, dans lequel chacune des lumières du corps interne (104) présente une section transversale circulaire.

7. Système de cathéter (10) selon la revendication 6, dans lequel la première lumière (114) présente un diamètre intérieur supérieur au diamètre intérieur de l'une quelconque des deuxième et troisième lumières (116, 118).

8. Système de cathéter (10) selon l'une quelconque des revendications précédentes, dans lequel :
lorsque la gaine externe (92) est disposée sur le ou les tubes expansibles (96), lesdits tubes expansibles (96) sont dans un état contracté, et
lorsque la gaine externe (92) est déplacée proximalement par rapport au corps (18a) pour faire apparaître les tubes expansibles (96), lesdits tubes expansibles (96) subissent une expansion pour prendre un état d'expansion dans lequel les tubes expansibles (96) présentent une dimension transversale maximale non contrainte (D2) qui est supérieure à une dimension transversale maximale du corps (18a) ou de la gaine externe (92).

9. Système de cathéter (10) selon la revendication 8, dans lequel, lorsque les tubes expansibles (96) sont à l'état contracté, au moins une partie desdits tubes expansibles (96) est radialement plus proche du corps (18a) que lorsque les tubes expansibles (96) sont à l'état d'expansion.

10. Système de cathéter (10) selon la revendication 8 ou 9, dans lequel le ou les tubes expansibles (96) prennent, à l'état d'expansion, une forme choisie parmi les suivantes : une forme hélicoïdale, en spirale, sphérique, conique, en fleur, en cage ou en panier.

11. Système de cathéter (10) selon l'une quelconque des revendications 8 à 10, dans lequel la dimension transversale maximale non contrainte (D2) est égale à au moins 150 %, éventuellement à au moins 300 %, de la dimension transversale maximale du corps (18a).

12. Système de cathéter (10) selon l'une quelconque des revendications précédentes, dans lequel le ou les tubes expansibles (96) comprennent un alliage à mémoire de forme.

13. Système de cathéter (10) selon la revendication 12, dans lequel l'alliage à mémoire de forme comprend du nitinol.

14. Système de cathéter (10) selon la revendication 12 ou 13, dans lequel le ou les tubes expansibles (96) comprennent en outre un polymère.

15. Système de cathéter (10) selon l'une quelconque des revendications précédentes, dans lequel le ou les tubes expansibles (96) sont conçus pour disperser du fluide dans plusieurs directions.
